# EUROPEAN PATENT APPLICATION

(11) **EP 2 774 722 A1**
(43) Date of publication of application: **10.09.2014**
(21) Application number: 12845990.6
(22) Date of filing: 16.10.2012
(51) Int. Cl.: B24C 1/06, A61L 17/00, A61L 27/00, B24C 11/00, C04B 41/91, C08J 9/00, C08J 9/36, C22C 14/00

(54) **MATERIAL HAVING PORES ON SURFACE, AND METHOD FOR MANUFACTURING SAME**

(30) Priority: 04.11.2011 JP 2011242912
(71) Applicant: Kyoto University, Kyoto-shi, Kyoto 606-8501 (JP)
(72) Inventor: YAO, Takeshi, Kyoto-shi Kyoto 606-8501 (JP); YABUTSUKA, Takeshi, Kyoto-shi Kyoto 606-8501 (JP)
(74) Representative: Sutcliffe, Nicholas Robert
(86) International application number: PCT/JP2012/076738
(87) International publication number: WO 2013/065476

(57) **Abstract**

This invention makes it possible to impart bioactivity to a variety of materials by sandblasting to form pores in the surface thereof.

## Description

### Technical Field

The present invention relates to a material having pores in the surface, and a method for manufacturing the same.

### Background Art

Conventionally, various biologically active ceramics have been developed; however, the mechanical properties of such ceramics differ greatly from those of *in vivo* bone. This limits the sites in which they can actually be used. In order to improve the mechanical properties, the use of an organic polymer material, metal material, or the like is desirable. However, most of such materials to which bioactivity has been directly imparted have not yet been practically applied.

In recent years, the development of bioactive organic polymer materials has advanced, resulting in the commercial availability of HAPEX (a registered trademark), a material to which bioactivity is imparted by kneading a high-density polyethylene with apatite. Furthermore, in recent years, a material called AHFIX (a registered trademark), to which bioactivity has been imparted by subjecting pure titanium to an alkali heat treatment, is being used in the joint portion of an artificial hip prosthesis. In HAPEX (a registered trademark), due to a large amount of apatite, good flexibility, which is an inherent property of organic polymer materials, tends to be undermined. Furthermore, HAPEX (a registered trademark) does not have satisfactory adhesion strength (about 3 MPa) to apatite layers generated *in vivo* (Non-Patent Literature 1). In addition, because the mechanical strength of pure titanium alone is unsatisfactory for use as an artificial hip prosthesis, a method for directly imparting bioactivity to metal materials having high strength, such as titanium alloys and the like, is in demand. However, none of such metal materials other than pure titanium to which bioactivity is directly imparted has been practically applied.

### Citation List

### Non-Patent Literature

Non-patent Literature 1:
J.A. Juhasz, S.M. Best, M. Kawashita, N. Miyata, T. Kokubo, T. Nakamura, W. Bonfield, Bonding strength of the apatite layer formed on glass-ceramic apatite-wollastonite-polyethylene composites. J. Biomed. Mater. Res., 67A, 952-959 (2003)

### Summary of Invention

### Technical Problem

A problem to be solved by the present invention is to impart bioactivity to various materials. More specifically, a problem to be solved by the present invention is to provide various materials having pores in the surfaces thereof suitable for being treated to obtain bioactivity.

### Solution to Problem

The present inventors conducted extensive research and found that a material suitable for being treated to obtain bioactivity can be obtained by subjecting a hard material to sandblasting to form pores in the surface.

The present inventors conducted further extensive research and found that a material obtainable by the following method is extremely suitable for imparting bioactivity. That is, the method comprises a step of sandblasting a hard material to form pores in the surface thereof, wherein the method comprises a plurality of sandblasting steps, and includes a step of sandblasting using media having a particle size that is different from that used in the immediately preceding step. The present invention has been accomplished based on this finding and further study. More specifically, the present invention provides the following:
[Item I]
   A material having pores in the surface, the material being obtainable by a method comprising sandblasting a hard material to form pores in the surface thereof.
[Item II]
   The material according to Item I, wherein the particle size of media used in sandblasting is 1 to 50 µm.
[Item 1]
   The material according to Item I, wherein the method for forming pores comprises a plurality of sandblasting steps comprising:
   (A) sandblasting with media having a particle size that is different from that used in the immediately preceding sandblasting step.
[Item 2]
   The material according to Item 1, wherein Step (A) is performed 1 to 3 times.
[Item 3]
   The material according to Item 1 or 2, wherein at least one of the sandblasting steps performed as Step (A) and that performed immediately preceding Step (A) are performed with media having a particle size of 1 to 50 µm.
[Item 4]
   The material according to any one of Items 1 to 3, wherein the particle size of the media used in Step (A) is 1.5 to 10 times or 0.1 to 0.67 times that of the media used in the sandblasting step immediately preceding Step (A).
[Item 5]
   A material having pores in the surface, the material being obtainable by a method for forming pores in the surface by sandblasting a hard material, the method comprising:
   (1) sandblasting a hard material; and
   (2) sandblasting the hard material that was sandblasted in Step (1) with media having a particle size different from that of Step (1).
[Item 6]
   The material according to Item 5, wherein the particle size of the sandblasting media used in Step (1) and Step (2) is each 1 to 50 µm.
[Item 7]
   The material according to Item 5 or 6, wherein the particle size of the media used in Step (2) is 1.5 to 10 times or 0.1 to 0.67 times that of the media used in Step (1).
[Item 8]
   The material according to any one of Items 5 to 7, which is obtainable by a method further comprising:
   (3) sandblasting the hard material that was sandblasted in Step (2) with media having a particle size different from that of Step (2).
[Item 9]
   The material of any one of Items 5 to 8, wherein the particle size of the media used in Step (3) is 1 to 50 µm.
[Item 10]
   The material according to any one of Items 5 to 9, wherein the particle size of the media used in Step (3) is 1.5 to 10 times or 0.1 to 0.67 times that of the media used in Step (2).
[Item 11]
   The material according to any one of Items 1 to 10, wherein the sandblasting is performed using, as an abrasive, silicon carbide, alumina, diamond, or silica.
[Item 12]
   The material according to any one of Items 1 to 11, wherein the hard material is an organic polymer, a metal, a ceramic, or a complex thereof.
[Item 13]
   The material according to any one of Items 1 to 12, which is used as a medical or dental material.
[Item 14]
   The material according to any one of Items 1 to 13, wherein calcium phosphate compound-containing fine particles are adhered to the surface thereof.
[Item 15]
   The material according to any one of Items 1 to 13, which supports calcium phosphate compound-containing fine particles on a surface.
[Item 16]
   The material according to Item 14, wherein the method further comprises:
   (B) contacting the material obtained in Step (A) to a solution comprising calcium ions and hydrogen phosphate ions; and
   (C) precipitating calcium phosphate compound-containing fine particles in the surface of the material obtained in Step (B).
[Item III]
   A method for producing a material having pores in the surface thereof by subjecting a hard material to sandblasting.
[Item IV]
   The method according to Item III, wherein the particle size of the media is 1 to 50 µm.
[Item 17]
   The method according to Item III, which comprises a plurality of sandblasting steps, and further comprises:
   (A) sandblasting step using media having a particle size that is different from that used in the immediately preceding sandblasting step.

### Advantageous Effects of Invention

The use of the present invention can impart bioactivity to various materials.

### Brief Description of Drawings

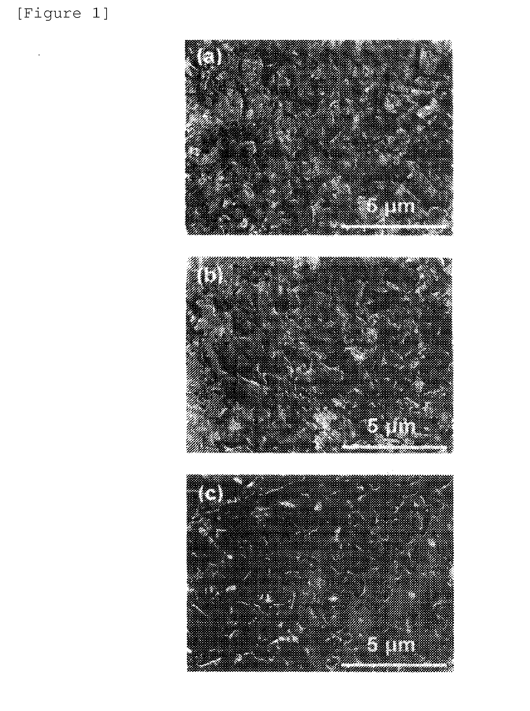

Fig. 1: SEM analysis images of (a) a titanium alloy SH porous surface material obtained by single-step sandblasting, (b) a titanium alloy IH porous surface material obtained by single-step sandblasting, and (c) a PET porous surface material obtained by single-step sandblasting, each with apatite nuclei precipitated in the pores.

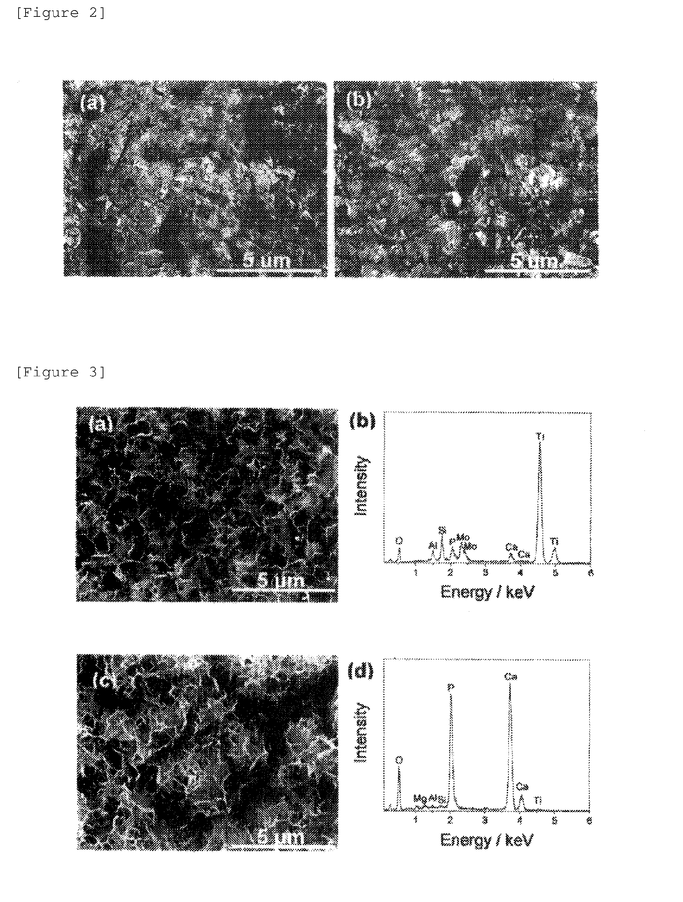

Fig. 2: SEM analysis images of (a) a titanium alloy porous surface material and (b) a PET porous surface material, each obtained by two-step sandblasting performed after initial sandblasting.
Fig. 3: (a) An SEM analysis image and (b) EDX profile of a titanium alloy SH porous surface material with apatite nuclei precipitated in the pores obtained by single-step sandblasting, after being immersed in SBF for one day, and (c) an SEM analysis image and (d) EDX profile of an SH porous surface material with apatite nuclei precipitated in the pores obtained by single-step sandblasting, after being immersed in SBF for seven days.

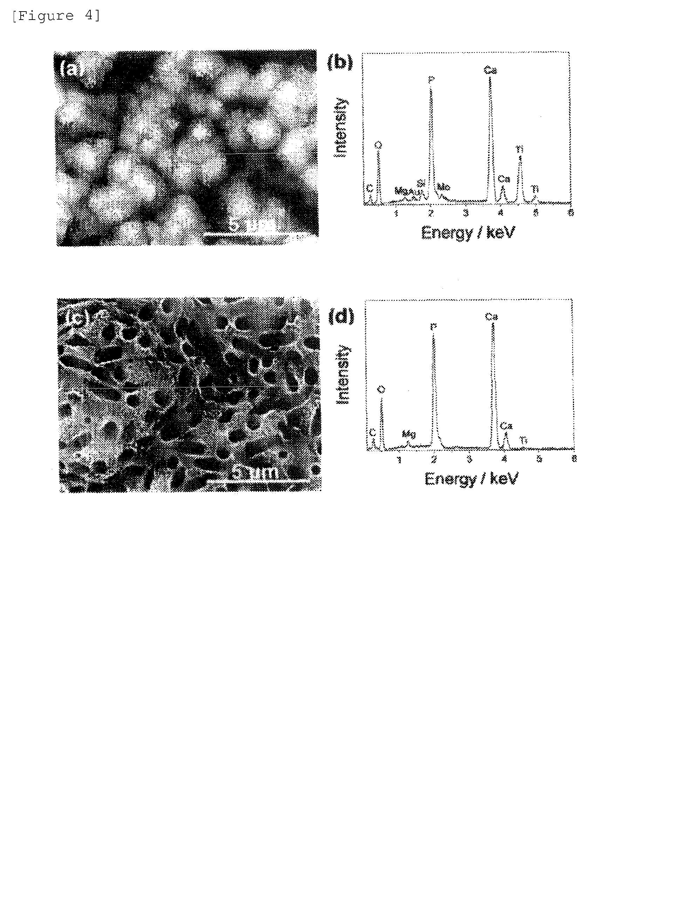

Fig. 4: (a) An SEM analysis image and (b) EDX profile of a titanium alloy IH porous surface material with apatite nuclei precipitated in the pores obtained by single-step sandblasting, after being immersed in SBF for one day, and (c) an SEM analysis image and (d) EDX profile of a titanium alloy SH porous surface material with apatite nuclei precipitated in the pores obtained by single-step sandblasting, after being immersed in SBF for seven days.

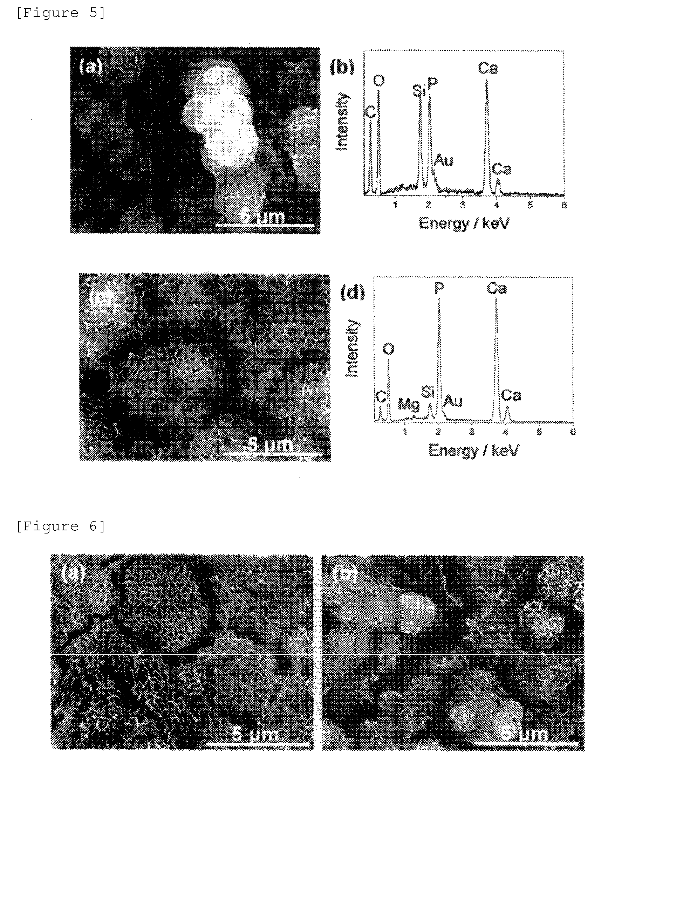

Fig. 5: (a) An SEM analysis image and (b) EDX profile of a PET porous surface material with apatite nuclei precipitated in the pores obtained by single-step sandblasting, after being immersed in SBF for one day, and (c) an SEM analysis image and (d) EDX profile of a titanium alloy PET porous surface material with apatite nuclei precipitated in the pores obtained by single-step sandblasting, after being immersed in SBF for seven days.
Fig. 6: (a) An SEM analysis image of a titanium alloy porous surface material with apatite nuclei precipitated in the pores obtained by two-step sandblasting and (b) an SEM analysis image of a PET porous surface material obtained by two-step sandblasting, each after being immersed in SBF for one day.

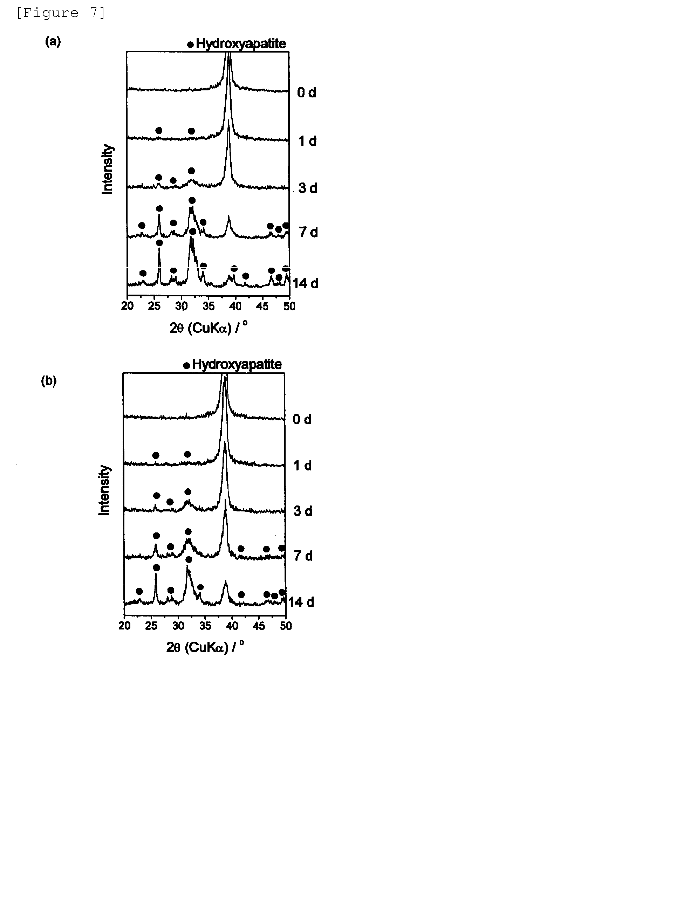

Fig. 7: TF-XRD profiles of (a) a titanium alloy SH porous surface material with apatite nuclei precipitated in the pores obtained by single-step sandblasting, and (b) a titanium alloy IH porous surface material with apatite nuclei precipitated in the pores obtained by two-step sandblasting, each after being immersed in SBF for 1 to 14 days.

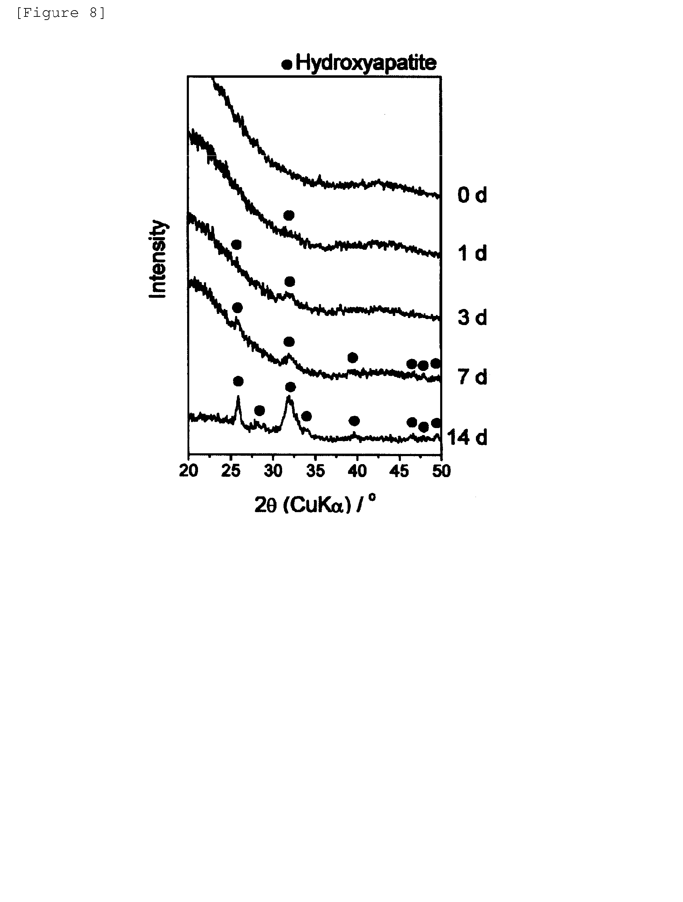

Fig. 8: A TF-XRD profile of a PET porous surface material with apatite nuclei precipitated in the pores obtained by single-step sandblasting, after being immersed in SBF for 1 to 14 days.

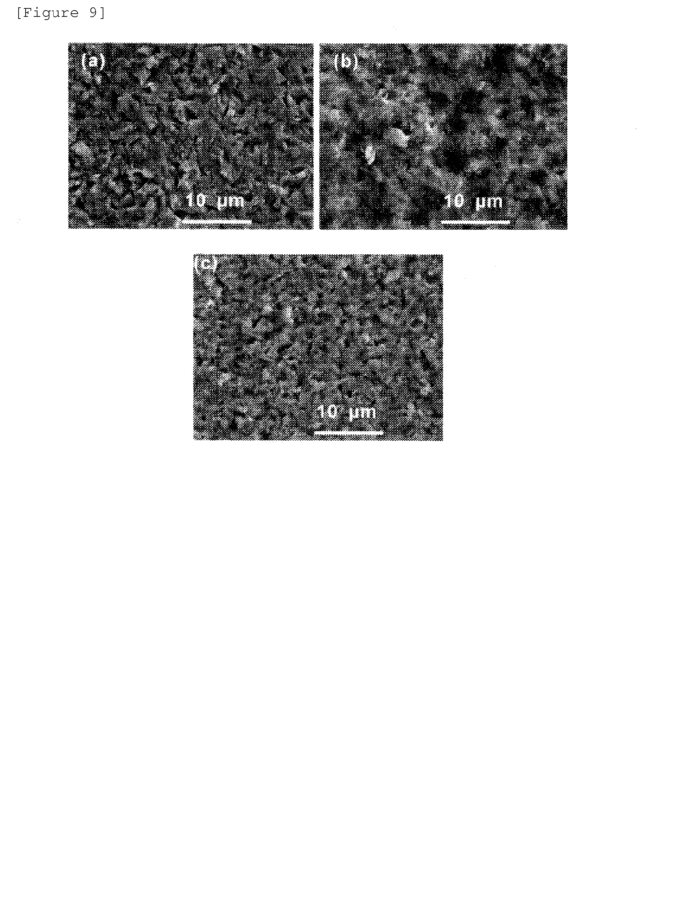

Fig. 9: SEM analysis images of (a) a titanium metal porous surface material, (b) a Ti-15Mo-5Zr-3Al porous surface material, and (c) a Ti-12Ta-9Nb-3V-6Zr-O porous surface material, each obtained by two-step sandblasting.

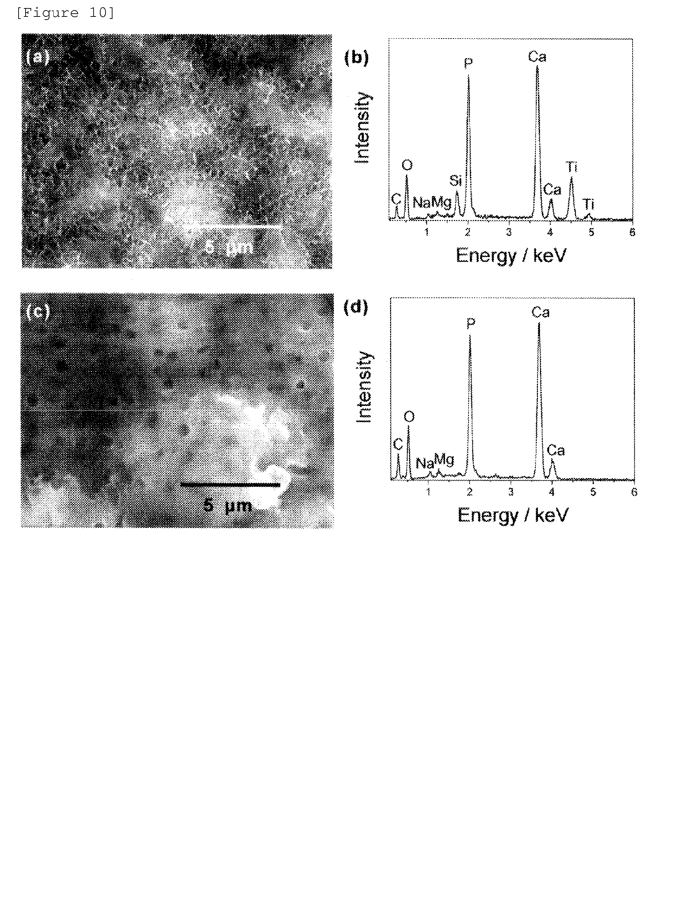

Fig. 10 (a) An SEM analysis image and (b) EDX profile of a titanium metal porous surface material with apatite nuclei precipitated in the pores after being immersed in SBF for one day, and (c) an SEM analysis image and (d) EDX profile of a titanium metal porous surface material with apatite nuclei precipitated in the pores after being immersed in SBF for seven days.

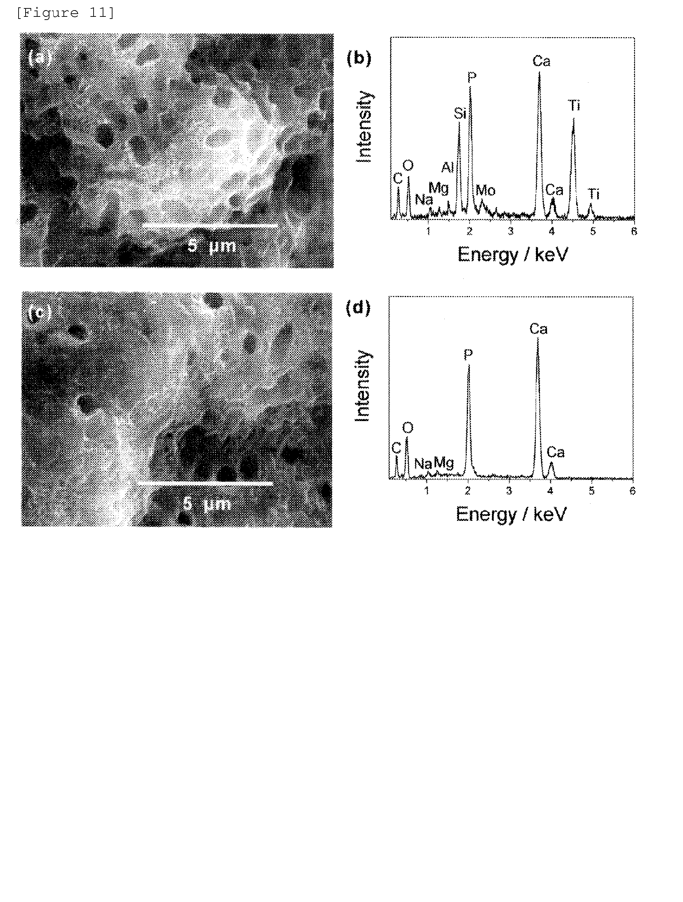

Fig. 11: (a) An SEM analysis image and (b) EDX profile of an apatite nuclei precipitated Ti-15Mo-5Zr-3Al porous surface material after being immersed in SBF for one day, and (c) an SEM analysis image and (d) EDX profile of Ti-15Mo-5Zr-3Al porous surface material with apatite nuclei precipitated in the pores, after being immersed in SBF for 7 days.

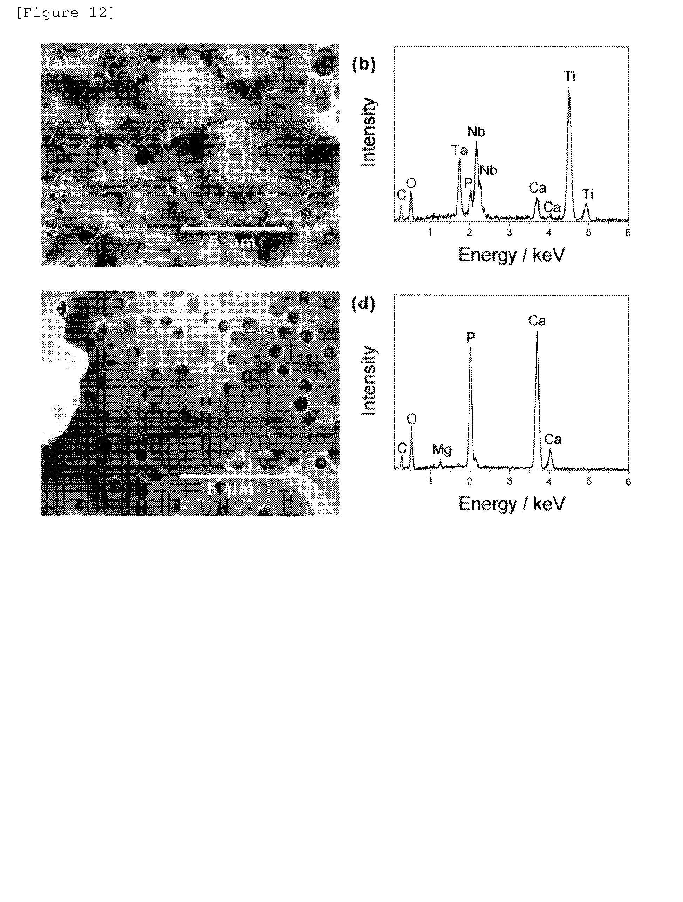

Fig. 12: (a) An SEM analysis image and (b) EDX profile of a Ti-12Ta-9Nb-3V-6Zr-O porous surface material with apatite nuclei precipitated in the pores, after being immersed in SBF for one day, and (c) an SEM analysis image and (d) EDX profile of a Ti-12Ta-9Nb-3V-6Zr-O porous surface material with apatite nuclei precipitated in the pores, after being immersed in SBF for 7 days.

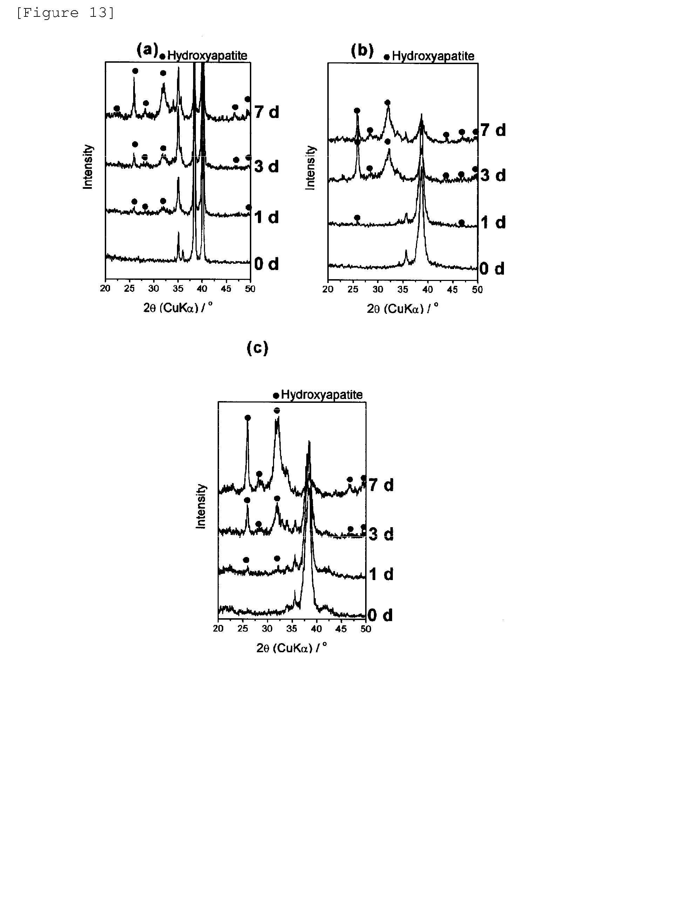

Fig. 13: TF-XRD profiles of (a) a titanium metal porous surface material with apatite nuclei precipitated in the pores, (b) a Ti-15Mo-5Zr-3Al porous surface material, and (c) a Ti-12Ta-9Nb-3V-6Zr-O porous surface material, each after being immersed in SBF for 1 to 7 days.

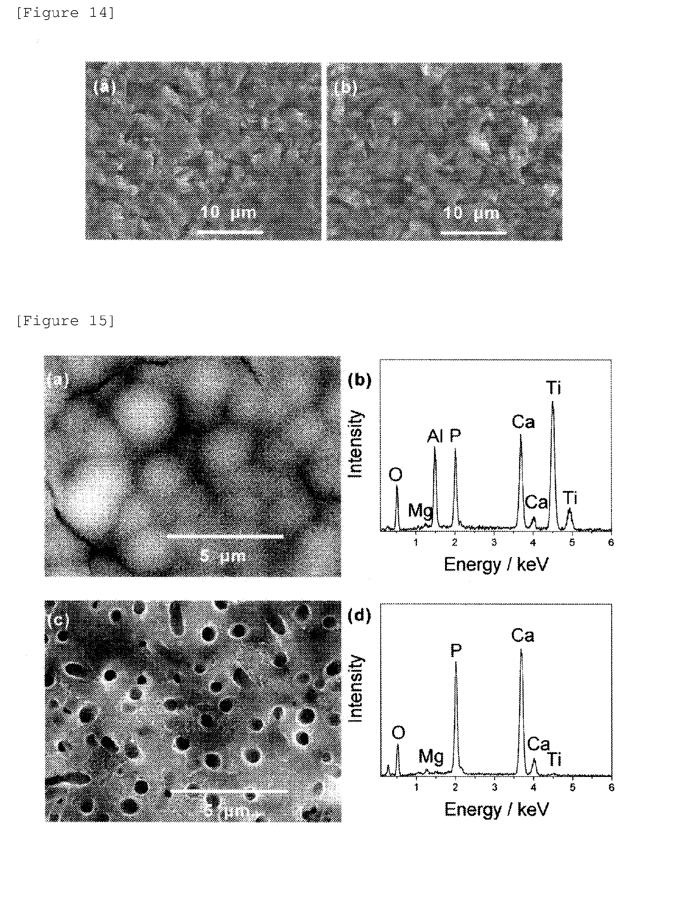

Fig. 14: SEM analysis images of (a) a Ti-6Al-4V porous surface material, and (b) a Ti-22V-4Al porous surface material, each obtained by two-step sandblasting.
Fig. 15: (a) An SEM analysis image and (b) EDX profile of aTi-6Al-4V porous surface material with apatite nuclei precipitated in the pores, after being immersed in SBF for one day, and (c) an SEM analysis image and (d) EDX profile of a Ti-6Al-4V porous surface material with apatite nuclei precipitated in the pores, after being immersed in SBF for 7 days.

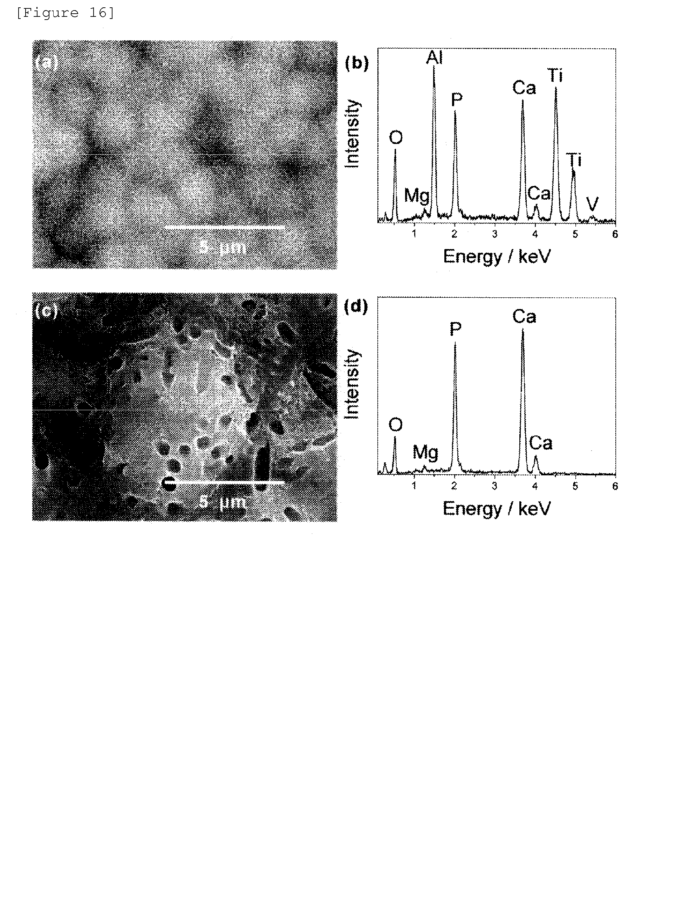

Fig. 16: (a) An SEM analysis image and (b) EDX profile of an apatite nuclei precipitated Ti-22Al-4V porous surface material after being immersed in SBF for one day, and (c) an SEM analysis image and (d) EDX profile of a Ti-22Al-4V porous surface material with apatite nuclei precipitated in the pores, after being immersed in SBF for 7 days.

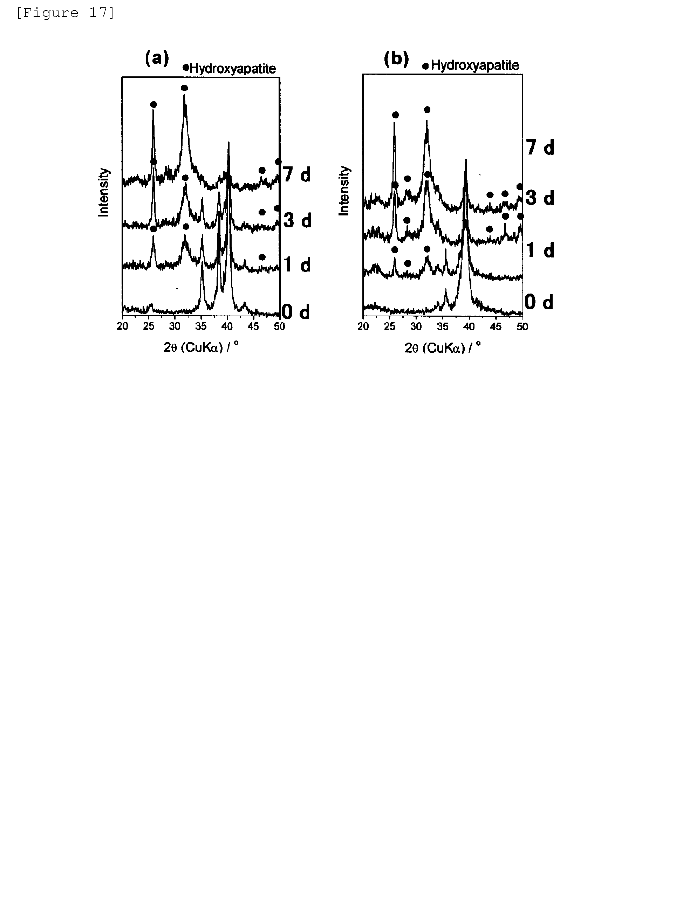

Fig. 17: TF-XRD profiles of (a) a Ti-6Al-4V porous surface material with apatite nuclei precipitated in the pores, and (b) a Ti-22V-4Al porous surface material, each after being immersed in SBF for 1 to 7 days.

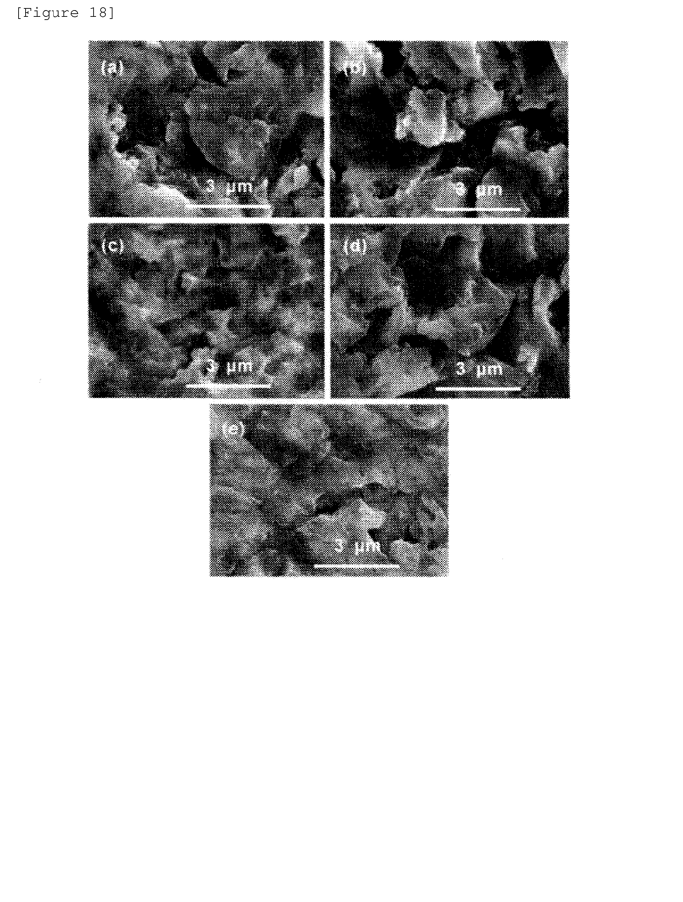

Fig. 18: SEM analysis images of (a) TMZR14, (b) TMZA14-14, (c) TMZA14-3, (d) TMZA3-14 and (e) TMZA3-3, each obtained by two-step sandblasting.

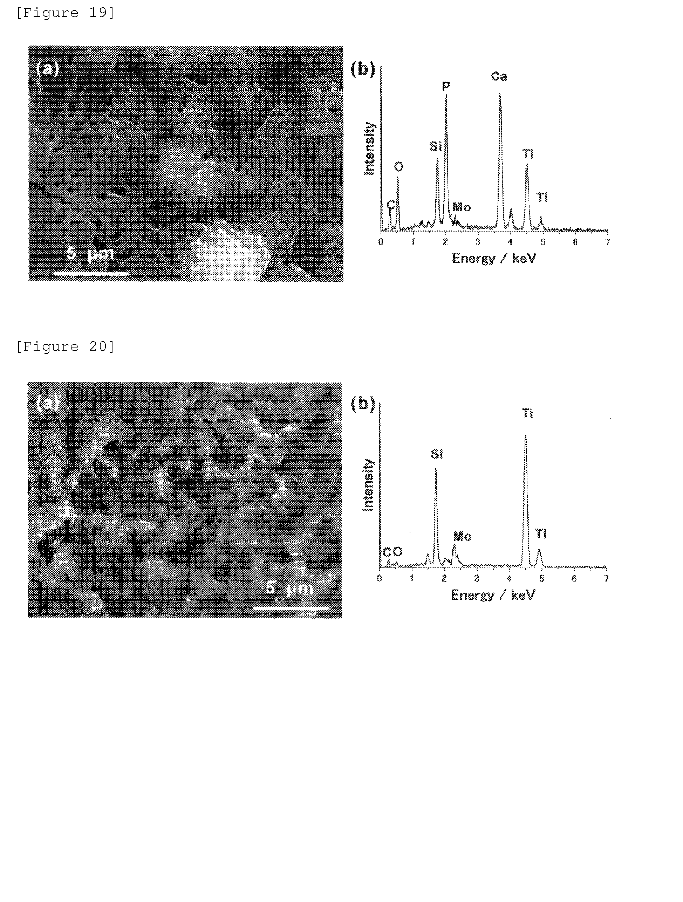

Fig. 19: (a) An SEM analysis image and (b) EDX profile of TMZA14 after being immersed in SBF for one day.
Fig. 20: (a) An SEM analysis image and (b) EDX profile of TMZA14-14 after being immersed in SBF for one day.

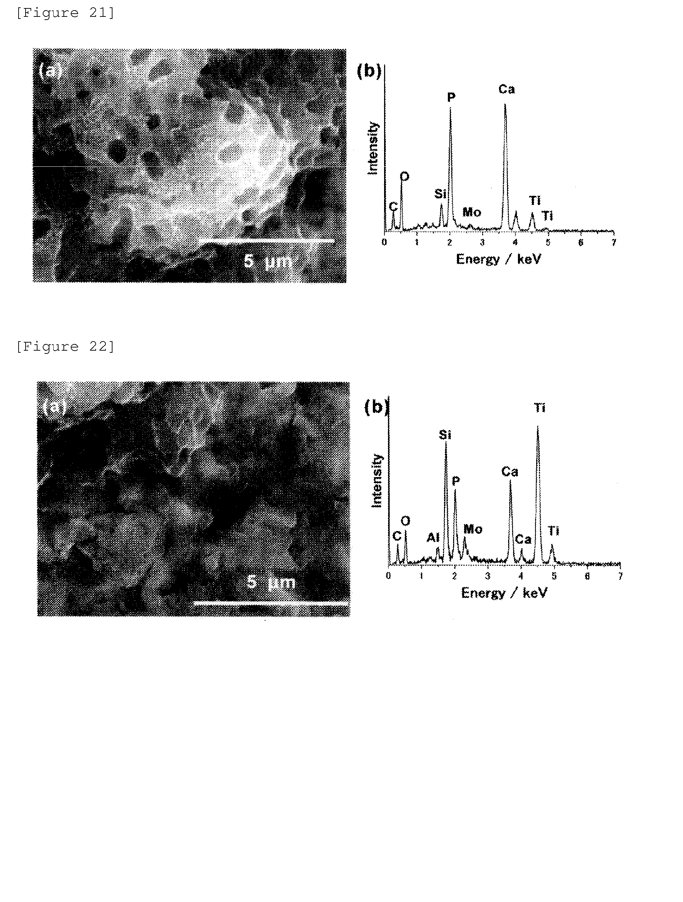

Fig. 21: (a) An SEM analysis image and (b) EDX profile of TMZA14-3 after being immersed in SBF for one day.
Fig. 22: (a) An SEM analysis image and (b) EDX profile of TMZA3-14 after being immersed in SBF for one day.

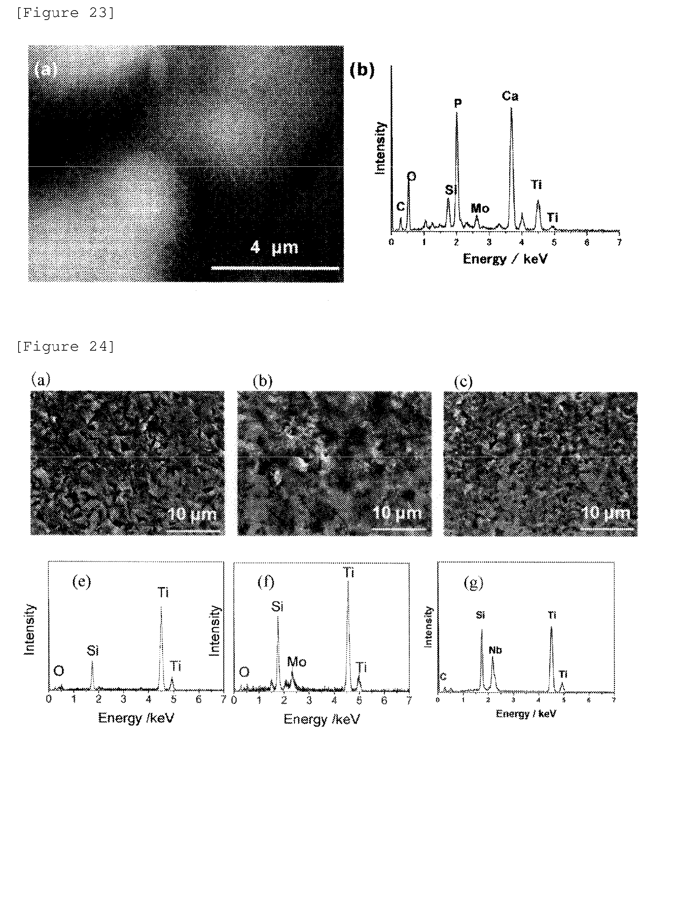

Fig. 23: (a) An SEM analysis image and (b) EDX profile of TMZA3-3 after being immersed in SBF for one day.
Fig. 24: SEM analysis images of (a) a titanium metal porous surface material, (b) a Ti-15Mo-5Zr-3Al porous surface material, and (c) a Ti-12Ta-9Nb-3V-6Zr-O porous surface material, each having calcium phosphate compound-containing fine particles precipitated in the pores; and EDX analysis results of (e) a titanium metal porous surface material, (f) a Ti-15Mo-5Zr-3Al porous surface material, and (g) a Ti-12Ta-9Nb-3V-6Zr-O porous surface material, each having calcium phosphate compound-containing fine particles precipitated in the pores.

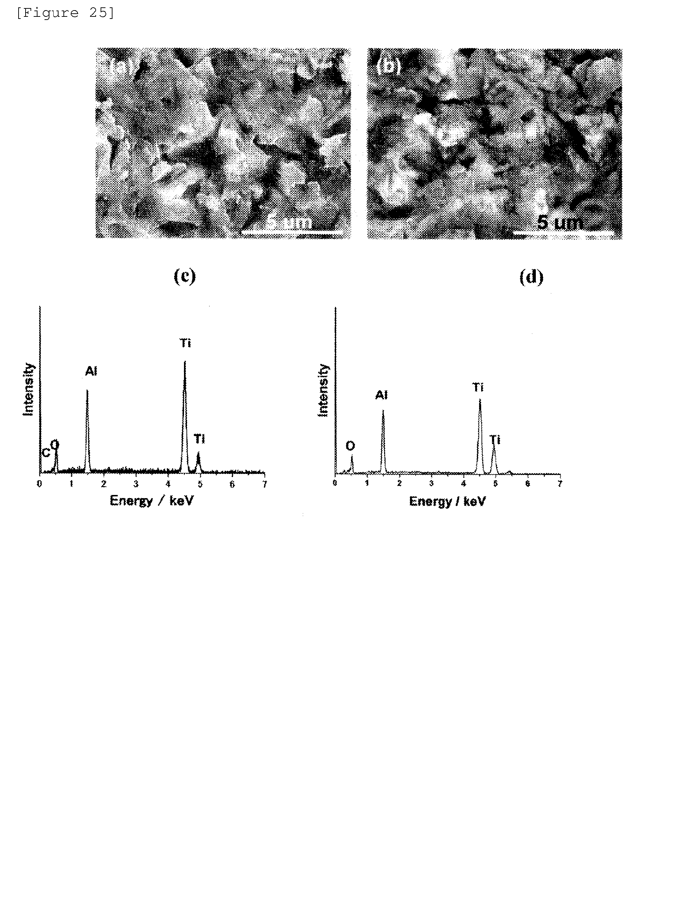

Fig. 25: SEM analysis images of (a) a Ti-6Al-4V porous surface material and (b) a Ti-22V-4Al porous surface material, each having calcium phosphate compound-containing fine particles precipitated in the pores; and EDX analysis results of (c) a Ti-6Al-4V porous surface material and (d) a Ti-22V-4Al porous surface material, each having calcium phosphate compound-containing fine particles precipitated in pores.

### Description of Embodiments

### 1. A method for producing a material having pores in the surface

The method for producing a material having pores in the surface of the present invention is a method of sandblasting a hard material to form pores in the surface.

The method for producing a material having pores in the surface of the present invention preferably comprises a plurality of sandblasting steps, and preferably comprises Step (A) that uses media having a particle size that is different from that of the sandblasting step immediately preceding Step (A).

The material having pores in the surface of the present invention has a large number of pores in the surface. In this specification, the material having pores in the surface of the present invention may be referred to as "a porous surface material."

The pores are not particularly limited. For example, the pores have a diameter of 100 µm or less.

The hard material as the raw material is not particularly limited. Examples thereof include organic polymers, metals, and ceramics. The hard material as the raw material may be, for example, a complex of at least two members selected from the group consisting of organic polymers, metals, and ceramics.

Examples of organic polymers include, but are not particularly limited to, polyethylene, polypropylene, polyethylene terephthalate, polyvinyl alcohol, ethylene-vinyl alcohol copolymer, polyether sulfone, polycaprolactone, and polylactic acid. Examples of organic polymers also include natural products, such as collagen. These organic polymers may be used solely or in a combination of two or more.

Examples of metals include, but are not particularly limited to, titanium and silver. These metals may be used solely (such as pure titanium) or in a combination of two or more. Examples of materials made of multiple kinds of metals include, but are not particularly limited to, alloys. Examples of alloys include, but are not particularly limited to, titanium alloy, stainless steel, and cobalt-chromium alloy. The titanium alloy is not particularly limited insofar as it is an alloy containing titanium as a major component. Examples thereof include Ti-15Mo-5Zr-3Al, Ti-6Al-4V, Ti-12Ta-9Nb-3V-6Zr-O and Ti-22V-4Al.

Examples of ceramics include, but are not particularly limited to, alumina, zirconia, silica gel, glass, glass ceramics and bone cement.

Organic polymers and metals, in particular, polyethylene terephthalate and titanium alloy, are preferable as the hard material used as the raw material.

The present invention enables the production of a material suitable for being treated to obtain bioactivity from various hard materials. For example, a method of treating titanium with sulfuric acid, thereby forming pores in the surface, is known. Regarding this method, the present inventors found that it is difficult to impart bioactivity to, for example, titanium alloy, stainless-steel, or the like, by this treatment. The present invention is capable of imparting bioactivity also to these hard materials. This is an advantage of the present invention.

The frequency of sandblasting treatments is not particularly limited. For example, sandblasting may be performed 2 to 5 times, or 2 to 4 times.

Among multiple sandblasting steps, Step (A) corresponds to the second or later sandblasting. The "immediately preceding sandblasting" (which hereinafter may also be referred to as "Step (a)") means sandblasting performed immediately preceding Step (A), among the multiple sandblasting steps.

Step (A) may be performed as many times as necessary. For example, when Step (A) is performed twice, the first Step (A) (which hereinafter may also be referred to as Step (A1), and sandblasting immediately preceding Step (A1) may be referred to as "Step (a1)") corresponds to the second or later sandblasting step, and the second Step (A) (which hereinafter may also be referred to as "Step (A2)"; and sandblasting immediately preceding Step (A2) may be referred to as "Step (a2)") corresponds to a step of further sandblasting a hard material that has been sandblasted in Step (A1) or a later sandblasting step. Therefore, when Step (A) is performed twice, the method for producing a porous surface material of the present invention includes at least Step (a1), Step (A1), and Step (A2) in this order; in other words, the method for producing a porous surface material of the present invention performs at least three sandblasting steps. In this case, the sandblasting (Step (a2)) immediately preceding Step (A2) is not always Step (A1) and may be a different sandblasting step performed after Step (A1). In this case, the method for producing a porous surface material of the present invention includes at least Step (a1), Step (A1), Step (a2), and Step (A2) in this order; in other words, the method for producing a porous surface material of the present invention performs at least four sandblasting steps. By performing Step (A) at least once, it is possible to obtain a porous surface material suitable for being treated to obtain bioactivity.

When Step (A) is performed once, the method for producing a porous surface material of the present invention includes the following method.

A method for sandblasting a hard material, thereby forming pores in the surface of the hard material, including:
(1) a step of sandblasting a hard material; and
(2) a step of sandblasting the hard material sandblasted in Step (1) with media having a particle size that is different from the particle size of the media used in Step (1).

When Step (A) is performed twice, the method for producing a porous surface material of the present invention includes the following method.

The above method further including (3), a step of sandblasting the hard material sandblasted in Step (2) with media having a particle size that is different from the particle size of the media used in Step (2).

The method for producing a porous surface material of the present invention also includes a method that performs Step (A) three times, in the same manner as above.

Among the plurality of sandblasting steps, at least one step performed as Step (A), and a step (Step (a)) immediately preceding Step (A) are performed with media having a particle size of preferably 1 µm to 50 µm (weight average particle diameter), more preferably, 2 µm to 30 µm (weight average particle diameter), and further preferably 3 µm to 15 µm (weight average particle diameter). By thus performing at least one set of sandblasting (Step (a) and Step (A)) with media having the above particle size range, it is possible to obtain a porous surface material suitable for being treated to obtain bioactivity. Further, since the present invention performs sandblasting with media having a small particle size, the present invention is advantageous in that the mechanical strength of the hard material will not be impaired. The "weight average particle diameter" refers to a particle diameter defined as "JISR6001 Particle Size of a Grinding Stone," which is a particle diameter at a cumulative height of 50% in a particle diameter distribution obtained by the electrical resistance test method. The method for measuring this particle diameter (JISR6001) is defined as "Method for Testing the Particle Size of a Grinding Stone" (JISR6002). This method determines the volume distribution of particles using an electrical resistance test.

The "particle size different from the particle size of media used in the immediately preceding step" means a particle size smaller or larger than the particle size used in the immediately preceding step.

The "particle size different from the particle size of media used in the immediately preceding step" is preferably a particle size different from the particle size in the immediately preceding sandblasting step by a factor of 1.5 to 10 or by a factor of 0.1 to 0.67, more preferably by a factor of 2 to 7 or by a factor of 0.14 to 0.5, and further preferably by a factor of 3 to 5 or by a factor of 0.2 to 0.33.

The "particle size different from the particle size of media used in the immediately preceding step" is preferably a particle size smaller than the particle size in the immediately preceding sandblasting. Specifically, the "particle size different from the particle size of media used in the immediately preceding sandblasting step is preferably a particle size smaller than the particle size of media used in the immediately preceding sandblasting step by a factor of 0.1 to 0.67, more preferably by a factor of 0.14 to 0.5, and further preferably by a factor of 0.2 to 0.33.

The combination of a particle size used in the immediately preceding sandblasting step and a particle size different from that in the immediately preceding sandblasting step is, for example, but is not particularly limited to, 10 to 20 µm and 1 to 5 µm, and more preferably 12 to 16 µm and 2 to 4 µm.

Sandblasting can be performed with a device generally used for metal surface processing, or the like.

Examples of the abrasive used for sandblasting steps include, but are not particularly limited to, silicon carbide, alumina, diamond, and silica. Although it is not particularly limited, for example, silicon carbide or alumina may be used as an abrasive when titanium metal or titanium alloy is used as a hard material. Examples of the aforementioned titanium alloy include, but are not particularly limited to, Ti-15Mo-5Zr-3Al, Ti-6Al-4V, Ti-12Ta-9Nb-3V-6Zr-O and Ti-22V-4Al. The sandblasting conditions may be determined according to the characteristics of the hard material, or the like. Although it is not particularly limited, when, for example, titanium metal and titanium alloy (for example, Ti-15Mo-5Zr-3Al, Ti-12Ta-9Nb-3V-6Zr-O and Ti-22V-4Al, or the like) are used as a hard material, and silicon carbide or alumina is used as an abrasive, a porous surface material suitable for being treated to obtain bioactivity can be obtained by performing Step (a) and Step (A) under a pressure of 0.85 MPa. Examples of the general pressure range used for sandblasting steps include 0.55 to 0.85 MPa, although it also depends on the device used for the sandblasting.

The method for producing a porous surface material of the present invention may further include one or more additional steps. For example, the method may further include a step of sandblasting a hard material for another purpose, i.e., a purpose other than imparting bioactivity. Examples of such a purpose include imparting a non-slip property. Generally, sandblasting for such a purpose is performed with media having a particle size of 100 µm or more. For example, the method for producing a porous surface material of the present invention also includes a method employing a sandblasting step for imparting a non-slip property to a hard material, and a plurality of sandblasting steps for the purpose of the present invention.

However, the method for producing a porous surface material of the present invention does not include a step for substantially or completely removing the material used for sandblasting, such as a water-washing step. Therefore, the components of the surface of the porous surface material obtained by the method for producing a porous surface material of the present invention change after sandblasting. The change of the components after sandblasting may be determined by an EDX composition analysis of the surface by detecting peaks derived only from the sandblasting material.

The porous surface material obtainable by the method for producing a porous surface material of the present invention is characterized by its suitability for imparting bioactivity. This characteristic allows the porous surface material to be used as a medical or dental material. Examples of medical materials include a bone prosthesis, a joint prosthesis, osteosynthesis plates, osteosynthesis screws, a tendon prosthesis, a ligament prosthesis and a cartilage prosthesis. Examples of dental materials include an artificial tooth root and an artificial alveolar bone.

### 2. Material having Pores in the Surface (Porous Surface Material)

The porous surface material of the present invention is a material obtainable by the above-described method for producing a porous surface material of the present invention.

As a result of the sandblasting, the porous surface material of the present invention has a large number of pores with complicated shapes in the surface. Preferably, the porous surface material of the present invention is subjected to a plurality of sandblasting steps with media having different particle sizes so as to be provided with a large number of pores with complicated shapes in the surface. Owing to such a distinctive pore structure, the porous surface material of the present invention is characterized by its suitability for imparting bioactivity. More specifically, since the pores of the porous surface material of the present invention are oriented to various directions, when hydroxyapatite grows in the pores, an interlocking effect is generated between the porous surface material and hydroxyapatite, thereby strengthening the adhesion between them.

As described above, the surface components of the porous surface material of the present invention change after sandblasting. The change of the surface components after sandblasting may be determined by EDX composition analysis of the surface by detecting peaks derived only from the sandblasting material.

Owing to its characteristic suitability for imparting bioactivity, the porous surface material of the present invention may be used as a medical or dental material. Examples of medical materials include a bone prosthesis, a joint prosthesis, osteosynthesis plates, osteosynthesis screws, a tendon prosthesis, a ligament prosthesis and a cartilage prosthesis. Examples of dental materials include an artificial tooth root and an artificial alveolar bone.

The case below is an example of imparting bioactivity. By adhering calcium phosphate compound-containing fine particles to the pores having the above-described distinctive structure, it is possible to impart bioactivity similar to a biological bone or biological tooth to the porous surface material of the present invention. By transplanting this porous surface material of the present invention in which calcium phosphate compound-containing fine particles are adhered to the surface thereof into a living body, the calcium phosphate compound-containing fine particles induce hydroxyapatite, thereby generating an interlocking effect between the porous surface material of the present invention and the hydroxyapatite, thus strengthening the adhesion between them.

Examples of the method for producing a porous surface material of the present invention in which calcium phosphate compound-containing fine particles are adhered to the surface thereof include a method that includes:
a Step (B) of contacting the material obtained in the Step (A) to a solution comprising calcium ions and hydrogen phosphate ions; and
a Step (C) of precipitating calcium phosphate compound-containing fine particles in the surface of the porous surface material obtained in the Step (B).

The fine particles containing a calcium phosphate compound (which hereinafter may be referred to as "calcium phosphate compound-containing fine particles") precipitated in Step (C) may be any particles insofar as they serve as nuclei (which may also be referred to as "apatite nuclei" in the present specification) for forming and growing, on a porous surface material, a covering layer containing a calcium phosphate compound. The composition of the fine particles is not particularly limited. Examples of calcium phosphate compounds include apatites, such as primary calcium phosphate (Ca(H₂PO₄)₂), secondary calcium phosphate (CaHPO₄), tertially calcium phosphate (Ca₃(PO₄)₂), tetracalcium phosphate (Ca₄(PO₄)₂O), octacalcium phosphate (Ca₈H₂(PO₄)₆), or hydroxyapatite, and amorphous calcium phosphates. They may have crystallization water. The calcium phosphate compound-containing fine particles are preferably fine particles containing a hydroxyapatite as a major component. A hydroxyapatite is a compound represented by the chemical formula Ca₁₀(PO₄)₆(OH)₂. "Hydroxyapatite" also means a compound resulting from substitution and/or deletion of the constituting elements of a hydroxyapatite. Examples of compounds resulting from substitution and/or deletion of the constituting elements of a hydroxyapatite include, but are not particularly limited to, compounds resulting from substitution of a part of the elements or a group of a hydroxyapatite with a Group 1 element in the periodic table, such as Na or K, a Group 2 element in the periodic table, such as Mg, a Group 4 element in the periodic table, such as Ti, a Group 12 element in the periodic table, such as Zn, a Group 17 element in the periodic table, such as F or Cl, a group such as CO₃²⁻, HPO₄²⁻, or SO₄²⁻, or a rare earth metal element. Such elements or groups contained in these compounds are derived from elements or groups contained in a solution for precipitating the fine particles. More specifically, the solution used in Step (B) is suitably designed according to the desired composition of the calcium phosphate compound-containing fine particles. The solution is described later. The number average particle diameter of the fine particles is preferably 1 nm to 50 µm, more preferably 10 nm to 10 µm, and further preferably 50 nm to 1 µm. By specifying the number average particle diameter of the fine particles within the above range, the formation and the growth of the covering layer containing a calcium phosphate compound on the surface of the porous surface material may be effectively induced and promoted. The fine particles are crystalline particles or amorphous particles.

Examples of the solution containing calcium ions and hydrogen phosphate ions used in Step (B) for precipitating the calcium phosphate compound-containing fine particles include a solution that precipitates calcium phosphate compound-containing fine particles when the pH or temperature is increased; namely, a solution containing 0.02 mM to 25 mM calcium ions and 0.01 mM to 10 mM hydrogen phosphate ions and having pH of 4.0 to 8.0. The method for preparing such a solution is not particularly limited; the solution may be prepared by a suitable known method. "Phosphorous hydrogen ion" is a general name for phosphoric acids giving PO₄³⁻ in an aqueous solution, such as phosphoric acid (H₃PO₄), dihydrogen phosphate ion (H₂PO₄⁻), phosphorous hydrogen ion (HPO₄²⁻), and phosphate ion (PO₄³⁻), and condensed phosphoric acid generated by polymerization of two or more PO₄³⁻.

The concentration of calcium ions is preferably 0.2 mM to 20mM, and more preferably 1.2 mM to 5 mM. The concentration of hydrogen phosphate ions is preferably 0.1 mM to 8 mM, and more preferably 0.5 mM to 2 mM. In order to precipitate fine particles having superior biocompatibility, a simulated body fluid (SBF) that contains, in addition to calcium ions and hydrogen phosphate ions, sodium ions, potassium ions, magnesium ions, chloride ions, hydrogen carbonate ions, and sulfate ions, and has a concentration similar to the ion concentration of human blood plasma is preferably used as a solution for precipitating the fine particles. The concentration of sodium ions in the simulated body fluid is preferably 1.4 mM to 1420 mM, more preferably 14 mM to 1140 mM, and further preferably 70 mM to 290 mM. The concentration of potassium ions in the simulated body fluid is preferably 0.05 mM to 50 mM, more preferably 0.5 mM to 40 mM, and further preferably 2.5 mM to 10 mM. The concentration of magnesium ions in the simulated body fluid is preferably 0.01 mM to 15 mM, more preferably 0.1 mM to 12 mM, and further preferably 0.7 mM to 3 mM. The concentration of chloride ions in the simulated body fluid is preferably 1.4 mM to 1500 mM, more preferably 14.5 mM to 1200 mM, and further preferably 70 mM to 300 mM. The concentration of hydrogen carbonate ions in the simulated body fluid is preferably 0.04 mM to 45 mM, more preferably 0.4 mM to 36 mM, and further preferably 2 mM to 9 mM. The concentration of sulfate ions in the simulated body fluid is preferably 5.0×10⁻³ mM to 5 mM, more preferably 0.05 mM to 4 mM, and further preferably 0.2 mM to 1 mM. A simulated body fluid containing such inorganic ions at concentrations closer to those of a body fluid is called 1.0SBF. 1.0SBF has a sodium ion concentration of 142.0 mM, a potassium ion concentration of 5.0 mM, a magnesium ion concentration of 1.5 mM, a calcium ion concentration of 2.5 mM, a chloride ion concentration of 147.8 mM, a hydrogen carbonate ion concentration of 4.2 mM, a phosphorous hydrogen ion concentration of 1.0 mM, and a sulfate ion concentration of 0.5 mM. In the present specification, a simulated body fluid having an inorganic ion concentration greater than 1.0 SBF by a factor of x (x is a positive real number) is called xSBF. In the present invention, it is preferable to use 0.5SBF to 5.0SBF solutions for precipitating fine particles.

In Step (B), it is not always necessary to permeate the solution for precipitating calcium phosphate compound-containing fine particles into all of the pores of the porous surface material. However, in order to form or grow the covering layer containing a calcium phosphate compound on the surface of the porous surface material while ensuring the high adhesion strength due to the interlocking effect, it is necessary to precipitate the fine particles, which serve as the nuclei for forming or growing the covering layer, in at least a part of the pores near the surface of the porous surface material. To this end, it is necessary to permeate the solution for precipitating fine particles into at least a part of the pores near the surface of the porous surface material. This can be ensured, for example, by removing air bubbles from the pores by performing deaeration after immersing the porous surface material in the solution, or by pushing the solution into the pores by a pressure treatment using a cold isotropic pressing apparatus.

Step (C) of precipitating the calcium phosphate compound-containing fine particles from the solution is preferably performed, for example, by adding a pH regulator having a buffering ability, such as tris(hydroxymethyl)aminomethane or ammonia, to the solution so as to increase the pH value of the solution. By using a pH regulator having a buffering ability, the pH of the solution can be accurately adjusted. For example, when the solution used in Step (1) has a pH of 4.0 to 7.1, it is possible to efficiently precipitate fine particles having superior biocompatibility in the pores by increasing the pH to 7.2 to 9.0. To ensure the precipitation of fine particles, after the pH is increased, it is preferable to allow the solution containing the porous surface material immersed therein to sit for a predetermined time, for example, 1 to 30 hours. Further, it is preferable to increase the pH while keeping the solution at 35°C to 60°C, and then allowing the solution to sit.

Further, Step (C) of precipitating the calcium phosphate compound-containing fine particles from the solution may also be performed by increasing the temperature of the solution. To efficiently and securely precipitate fine particles having superior biocompatibility in the pores, it is preferable to increase the temperature of the solution by 20°C or more. For example, when the temperature of the solution is 40°C or less, the temperature of the solution is preferably increased to 60°C or more. As an example of the method for increasing the temperature, the porous surface material may be brought into contact with a heater so that the temperature of the solution in contact with the porous surface material increases. When the porous surface material is made of a metal, it is also possible to increase the temperature of the porous surface material by electromagnetic induction heating. By increasing the temperature of the porous surface material, the temperature of the solution in contact with the porous surface material selectively increases, thereby efficiently precipitating calcium phosphate compound-containing fine particles in the porous surface material.

By embedding the porous surface material of the present invention in which calcium phosphate compound-containing fine particles are adhered to the surface thereof in a living body, a covering layer containing a calcium phosphate compound, i.e., a bone tissue, is formed or grown on the surface of the porous surface material from the nuclei (fine particles) precipitated in the pores with a high adhesion strength. Further, in the porous surface material of the present invention in which calcium phosphate compound-containing fine particles are adhered to the surface thereof, the calcium phosphate compound-containing fine particles are precipitated also in the deep pores, i.e., the pores in a deep portion of the porous surface material. With this structure, the porous surface material of the present invention has a feature such that it maintains the bioactivity even after a treatment such as cutting, or shaping by cutting. The porous surface material of the present invention in which calcium phosphate compound-containing fine particles are adhered to the surface thereof may be embedded in a living body after forming or growing the covering layer containing a calcium phosphate compound on its surface using a simulated body fluid or the like. Further, by subjecting the porous surface material to a plasma surface treatment before Step (B) to impart a functional group, such as a hydroxyl group, to the surface, it is possible to form or grow a covering layer containing a calcium phosphate compound inside or outside a living body with a higher adhesion strength.

The porous surface material of the present invention in which calcium phosphate compound-containing fine particles are adhered to the surface thereof may be used as a medical or dental material. Examples of medical materials include a bone prosthesis, a joint prosthesis, osteosynthesis plates, osteosynthesis screws, a tendon prosthesis, a ligament prosthesis and a cartilage and prosthesis. Examples of dental materials include an artificial tooth root and an artificial alveolar bone.

### Examples

### Example 1: Production of porous surface materials by single-step sandblasting

Porous surface materials were produced by single-step sandblasting as follows. A titanium alloy plate with a size of 15×10×3 mm³ (Ti-15Mo-5Zr-3Al; Kobe Steel, Japan) and a polyethylene terephthalate (PET) plate with a size of 15×10×2 mm³ were used as hard materials. Sandblasting (PNEUMA-BLASTER (registered trademark) SFC-2; Fuji Manufacturing, Japan) was performed at a pressure of 0.85 MPa using a silicon carbide media having a weight average particle diameter of 3.0±0.4 µm (particle diameter at a cumulative height of 50% in particle diameter distribution obtained by the electrical resistance test method ("JISR6001 Particle Size of a Grinding Stone")) to form a large number of pores in the surfaces of the titanium alloy plate and PET plate. The thus-obtained titanium alloy plate in which pores were formed was washed with acetone and distilled water using an ultrasonic cleaner and dried at room temperature. The thus-obtained PET plate in which pores were formed was washed with ethanol and distilled water using an ultrasonic cleaner and dried at room temperature.

### Example 2: Production of porous surface materials by two-step sandblasting

Porous surface materials were produced by two-step sandblasting as follows. A titanium alloy plate with a size of 15×10×3 mm³ (Ti-15Mo-5Zr-3Al; Kobe Steel, Japan) and a polyethylene terephthalate (PET) plate with a size of 15×10×2 mm³ were used as hard materials. A first sandblasting step (PNEUMA-BLASTER (registered trademark) SFC-2; Fuji Manufacturing, Japan) was performed at a pressure of 0.85 MPa using a silicon carbide media having a weight average particle diameter of 14.0±1.0 µm (JISR6001 Particle Size of a Grinding Stone) to treat the surfaces of the titanium alloy plate and PET plate. Subsequently, a second sandblasting step was performed using a silicon carbide media having a weight average particle diameter of 3.0±0.4 µm (JISR6001 Particle Size of a Grinding Stone) in the same manner as in the first step to treat the surfaces of the titanium alloy plate and PET plate. The thus-obtained titanium alloy plate in which pores were formed by two-step sandblasting was washed with acetone and distilled water using an ultrasonic cleaner and dried at room temperature. The thus-obtained PET plate in which pores were formed by two-step sandblasting was washed with ethanol and distilled water using an ultrasonic cleaner and dried at room temperature.

### Example 3: Production of porous surface materials in which calcium phosphate compound-containing fine particles were adhered to the surfaces thereof

The porous surface materials obtained in Examples 1 and 2 were immersed in simulated body fluid (SBF) as described below, thereby producing porous surface materials in which calcium phosphate compound-containing fine particles were adhered to the surfaces thereof. The SBF was prepared as follows. Reagents NaCl, NaHCO₃, KCl, K₂HPO₄·3H₂O, MgCl₂·6H₂O, CaCl₂, and Na₂SO₄ were dissolved in ultrapure water with the composition shown in Table 1. Table 1 also shows ion concentrations in human blood plasma for reference.

**Table 1**

| | Ion Concentration/mmol·dm⁻³ | | | | | | | |
|---|---|---|---|---|---|---|---|---|
| | Na⁺ | K⁺ | Mg²⁺ | Ca²⁺ | Cl⁻ | HCO₃⁻ | HSO₄²⁻ | SO₄²⁻ |
| Human Blood Plasma | 142.0 | 5.0 | 2.5 | 1.5 | 103.8 | 27.0 | 1.0 | 0.5 |
| SBF | 142.0 | 5.0 | 2.5 | 1.5 | 147.8 | 4.2 | 1.0 | 0.5 |

The pH of the SBF was adjusted to pH 8.00 at 36.5°C using trishydroxymethylaminomethane. The titanium alloy porous surface materials obtained in Examples 1 and 2 were immersed in this SBF, and cold isostatic pressing (CIP-SI; Kobe Steel, Japan) was performed at a pressure of 392 MPa for 60 minutes to permeate the SBF into the pores of the porous surface materials. Subsequently, the SBF was heated at 60.0°C for 24 hours using a thermostatic chamber with the porous surface materials immersed in the SBF. Using this procedure, calcium phosphate compound-containing fine particles as apatite nuclei were precipitated in the pores of the titanium alloy porous surface materials. Hereafter, the titanium alloy porous surface materials thus heated may be referred to as "SH (solution heated) porous surface materials."

The pH of the SBF was adjusted to pH 8.10 at 36.5°C using trishydroxymethylaminomethane. Similar to the above, the titanium alloy porous surface materials obtained in Examples 1 and 2 were immersed in this SBF, and cold isostatic pressing (CIP-SI; Kobe Steel, Japan) was performed at a pressure of 392 MPa for 60 minutes to permeate the SBF in the pores of the porous surface materials. Subsequently, the porous surface materials in the SBF were directly heated by using electromagnetic induction at 3 kW for 30 minutes. Using this procedure, calcium phosphate compound-containing fine particles as apatite nuclei were precipitated in the pores of the titanium alloy porous surface materials. The thus-obtained porous surface materials were washed with distilled water and dried at room temperature. Hereafter, the titanium alloy porous surface materials thus heated may be referred to as "IH (induction heated) porous surface materials."

Further, the pH of the SBF was adjusted to pH 8.00 at 36.5°C using trishydroxymethylaminomethane. Similar to the above, the PET porous surface materials obtained in Examples 1 and 2 were immersed in this SBF, and cold isostatic pressing (CIP-SI; Kobe Steel, Japan) was performed at a pressure of 392 MPa for 60 minutes to permeate the SBF in the pores of the porous surface materials. Subsequently, the SBF was heated by microwave at 500 W for 3 minutes with the porous surface materials immersed in the SBF. Using this procedure, calcium phosphate compound-containing fine particles as apatite nuclei were precipitated in the pores of the PET porous surface materials. The thus-obtained porous surface materials were washed with distilled water and dried at room temperature.

### Test Example 1: Surface observation of porous surface materials

Each porous surface material (produced using titanium alloy or PET), in which calcium phosphate compound-containing fine particles were adhered to the surface thereof, obtained in Example 3 through Example 1 (single-step sandblasting) was observed using a scanning electron microscope (SEM; SU6600, Hitachi High-Technologies, Japan). Fig. 1 shows the results. The pores formed in the surface by sandblasting were observed. Apatite nuclei could not be confirmed by SEM, and no peaks of P or Ca were detected by EDX. This is assumed to be because the apatite nuclei were not sufficiently large.

The surface of the porous surface material (produced using titanium alloy or PET) obtained in Example 2 was observed using a scanning electron microscope (SEM; SU6600, Hitachi High-Technologies, Japan). Fig. 2 shows the results. The porous surface material obtained from the porous surface material produced by two-step sandblasting was confirmed to have a more complicated shape than those of the porous surface materials produced by single-step sandblasting.

### Test Example 2: Evaluation of the bioactivity of the porous surface material, in which calcium phosphate compound-containing fine particles were adhered to the surface thereof

1. The bioactivity of each porous surface material, in which calcium phosphate compound-containing fine particles were adhered to the surface thereof, obtained in Example 3 was evaluated as follows. Each of the porous surface materials, in which calcium phosphate compound-containing fine particles were adhered to the surface thereof, obtained in Example 3 was immersed in SBF (pH 7.40 at 36.5°C). The surface of each porous surface material having hydroxyapatite formed thereon was analyzed using thin film X-ray diffraction (TF-XRD; Rint 2500; Rigaku Corporation, Japan), a scanning electron microscope (SEM; SU6600, Hitachi High-Technologies, Japan), and energy-dispersive X-ray spectroscopy (EDX; XFlash (registered trademark) 5010; Bruker, U.S.A.).

### SEM and EDX observation results

Each porous surface material (produced using titanium alloy or PET), in which calcium phosphate compound-containing fine particles were adhered to the surface thereof, obtained in Example 3 through Example 1 (single-step sandblasting) was immersed in SBF for one day or seven days, followed by SEM observation. Figs. 3 to 5 show the results. After one-day immersion, the surface was confirmed to be entirely covered with needle-shaped hydroxyapatite crystals. After seven-day immersion, the SEM observation confirmed the growth of the hydroxyapatite crystals, and the EDX analysis confirmed an increase in the intensity of peaks of P and Ca. As shown in Fig. 4(c), pores were observed; however, these pores were actually formed due to the hydroxyapatite layer, and were not formed in the titanium alloy surface by sandblasting.

Each porous surface material (produced using titanium alloy or PET), in which calcium phosphate compound-containing fine particles were adhered to the surface thereof, obtained in Example 3 through Example 2 (two-step sandblasting) was immersed in SBF for one day, followed by SEM observation. Fig. 6 shows the results. The results confirm that the surface was entirely covered with needle-shaped crystals specific to hydroxyapatite. Peaks of P and Ca were detected by EDX. This indicates that hydroxyapatite induced from apatite nuclei was precipitated in the pores to cover the entire surface of the porous surface material within one day.

### Thin film X-ray diffraction results

Each porous surface material (produced using titanium alloy or PET), in which calcium phosphate compound-containing fine particles were adhered to the surface thereof, obtained in Example 3 through Example 1 (single-step sandblasting) was immersed in SBF for 1 to 14 days, followed by thin film X-ray diffraction analysis. Figs. 7 and 8 show the results. After one-day immersion, precipitation of hydroxyapatite was suggested. As the immersion period became longer, more peaks of hydroxyapatite were detected with higher intensity. The combination of the thin film X-ray diffraction results, SEM observation results, and EDX observation results suggest that hydroxyapatite was induced from apatite nuclei to cover the entire surface.

Each porous surface material, in which calcium phosphate compound-containing fine particles were adhered to the surface thereof, obtained in Example 3 through Example 2 (two-step sandblasting) was immersed in SBF for one day, followed by thin film X-ray diffraction analysis. As a result, peaks of hydroxyapatite were detected. This indicates that hydroxyapatite was induced after one-day immersion.

2. The adhesion strength between the porous surface material and hydroxyapatite formed on the surface thereof was measured using a modified ASTM C-633 method (W. Lacefield, Hydroxylapatite coatings, in: L.L. Hench, J. Wilson (Eds.), An Introduction to Bioceramics, World Sci. Singapore, 1993, pp. 223-238). Both surfaces of the porous surface material were adhered to stainless steel jigs (10 x 10 mm²) with an Araldite (registered trademark) adhesive, and a tensile load was applied using a universal testing machine (AGS-H Autograph; Shimazu Corporation, Japan) at a cross-head speed of 1 mm·mm⁻¹ until breakage.

The titanium alloy IH porous surface material, in which calcium phosphate compound-containing fine particles were adhered to the surface thereof, obtained in Example 3 through Example 2 (two-step sandblasting) had an adhesion strength of 14.6±3.5 MPa (the average of ten samples). In contrast, the titanium alloy SH porous surface material had an adhesion strength of 3.5±1.1 MPa (the average of ten samples), and the titanium alloy IH porous surface material had an adhesion strength of 5.7±1.1 MPa (the average of ten samples), each having calcium phosphate compound-containing fine particles adhered to the surface thereof and each being obtained in Example 3 through Example 1 (single-step sandblasting). The PET porous surface material, in which calcium phosphate compound-containing fine particles were adhered to the surface thereof, obtained in Example 3 through Example 2 (two-step sandblasting) had an adhesion strength of 6.2±2.2 MPa (the average of ten samples). In contrast, the PET porous surface material, in which calcium phosphate compound-containing fine particles were adhered to the surface thereof, obtained in Example 3 through Example 1 (single-step sandblasting) had an adhesion strength of 3.9±1.1 MPa (the average of ten samples). In both the titanium alloy porous surface materials and the PET porous surface materials, those obtained through two-step sandblasting had significantly higher adhesion strength, compared to those obtained through single-step sandblasting. This is assumed to be because pores with more complicated shapes were formed in the surface by two-step sandblasting, and a greater interlocking effect was thereby achieved between hydroxyapatite and the titanium alloy or PET.

### Example 4: Application to different types of metal

Porous surface materials were produced in the same manner as in the preceding Examples except that different types of metal were used.

Porous surface materials were produced by two-step sandblasting as follows. A titanium metal plate with a size of 15×10×2 mm³ (Kobe Steel, Japan), a Ti-15Mo-5Zr-3Al plate with a size of 15×10×3 mm³ (Kobe Steel, Japan), and a Ti-12Ta-9Nb-3V-6Zr-O plate with a size of 20×10×1 mm³ (Nissey, Japan) were used as metal materials. A first sandblasting step (PNEUMA-BLASTER (registered trademark) SFC-2; Fuji Manufacturing, Japan) was performed at a pressure of 0.85 MPa using a silicon carbide media having a weight average particle diameter of 14.0±1.0 µm (JISR6001 Particle Size of a Grinding Stone) to treat the surfaces of the titanium metal plate, Ti-15Mo-5Zr-3Al plate, and Ti-12Ta-9Nb-3V-6Zr-O plate. Subsequently, a second sandblasting step was performed using a silicon carbide media having a weight average particle diameter of 3.0±0.4 µm (JISR6001 Particle Size of a Grinding Stone) in the same manner as in the first step to treat the surfaces of the titanium metal plate, Ti-15Mo-5Zr-3Al plate, and Ti-12Ta-9Nb-3V-6Zr-O plate. The thus-obtained titanium metal plate, Ti-15Mo-5Zr-3Al plate, and Ti-12Ta-9Nb-3V-6Zr-O plate in which pores were formed were washed with acetone and distilled water using an ultrasonic cleaner and dried at room temperature.

### Example 5: Application to different types of metal

Apatite nuclei were precipitated on the porous surface materials obtained in Example 4.

The porous surface materials obtained in Example 4 were immersed in simulated body fluid (SBF) as described below, thereby producing porous surface materials having calcium phosphate compound-containing fine particles precipitated in pores. The SBF was prepared as follows. Reagents NaCl, NaHCO₃, KCl, K₂HPO₄·3H₂O, MgCl₂·6H₂O, CaCl₂, and Na₂SO₄ were dissolved in ultrapure water with the composition shown in Table 1 above.

The pH of the SBF was adjusted to pH 8.10 at 36.5°C using trishydroxymethylaminomethane. Similar to the above, the porous surface materials obtained in Example 4, i.e., the titanium metal porous surface material, the Ti-15Mo-5Zr-3Al porous surface material, and the Ti-12Ta-9Nb-3V-6Zr-O porous surface material, were immersed in this SBF, and cold isostatic pressing (CIP-SI; Kobe Steel, Japan) was performed at a pressure of 200 MPa for 60 minutes to permeate the SBF in the pores of the porous surface materials. Subsequently, the porous surface materials in the SBF were directly heated by using electromagnetic induction at 3 kW for 180 minutes. Using this procedure, calcium phosphate compound-containing fine particles as apatite nuclei were precipitated in the pores of the titanium metal porous surface material, the pores of the Ti-15Mo-5Zr-3Al porous surface material, and the pores of the Ti-12Ta-9Nb-3V-6Zr-O porous surface material. The thus-obtained porous surface materials were washed with distilled water and dried at room temperature.

### Test Example 3: Surface observation of porous surface materials

Each porous surface material (produced using titanium metal, Ti-15Mo-5Zr-3Al, or Ti-12Ta-9Nb-3V-6Zr-O), in which calcium phosphate compound-containing fine particles were precipitated in the pores, obtained in Example 5 through Example 4 (two-step sandblasting) was observed using a scanning electron microscope (SEM; SU6600, Hitachi High-Technologies, Japan). Fig. 9 shows the results. The pores formed in the surface by sandblasting were observed.

### Test Example 4: of the bioactivity of the porous surface material, in which calcium phosphate compound-containing fine particles were adhered to the surface thereof

The bioactivity of each porous surface material, in which calcium phosphate compound-containing fine particles were precipitated in the pores, obtained in Example 5 was evaluated as follows. Each porous surface material, in which calcium phosphate compound-containing fine particles were precipitated in the pores, obtained in Example 5 was immersed in SBF (pH 7.40 at 36.5°C). The surface of each porous surface material having hydroxyapatite formed thereon was analyzed using thin film X-ray diffraction (TF-XRD; Rint 2500; Rigaku Corporation, Japan), a scanning electron microscope (SEM; SU6600, Hitachi High-Technologies, Japan), and energy-dispersive X-ray spectroscopy (EDX; XFlash (registered trademark) 5010; Bruker, U.S.A.).

### SEM and EDX observation results

Each porous surface material (produced using titanium metal, Ti-15Mo-5Zr-3Al, or Ti-12Ta-9Nb-3V-6Zr-O), in which calcium phosphate compound-containing fine particles were precipitated in the pores, obtained in Example 5 through Example 4 was immersed in SBF for one day or seven days, followed by SEM observation. Figs. 10 to 12 show the results. After one-day immersion, the surface was confirmed to be entirely covered with needle-shaped hydroxyapatite crystals. After seven-day immersion, the SEM observation confirmed the growth of the hydroxyapatite crystals, and the EDX analysis confirmed an increase in the intensity of peaks of P and Ca. As shown in Figs. 10(c), 11(a), 11(c), and 12(c), pores were observed; however, these pores were actually formed due to the hydroxyapatite layer, and were not formed in the titanium alloy surface by sandblasting.

### Thin film X-ray diffraction results

Each porous surface material (produced using titanium metal, Ti-15Mo-5Zr-3Al, or Ti-12Ta-9Nb-3V-6Zr-O), in which calcium phosphate compound-containing fine particles were precipitated in the pores, obtained in Example 5 through Example 4 was immersed in SBF for 1 to 7 days, followed by thin film X-ray diffraction analysis. Fig. 13 shows the results. After one-day immersion, precipitation of hydroxyapatite was suggested. As the immersion period became longer, more peaks of hydroxyapatite were detected with higher intensity. The combination of the thin film X-ray diffraction results, SEM observation results, and EDX observation results suggest that hydroxyapatite was induced from apatite nuclei to cover the entire surface.

2. The adhesion strength between the porous surface material and hydroxyapatite formed on the surface thereof was measured using a modified ASTM C-633 method (W. Lacefield, Hydroxylapatite coatings, in: L.L. Hench, J. Wilson (Eds.), An Introduction to Bioceramics, World Sci. Singapore, 1993, pp. 223-238). Both surfaces of the porous surface material were adhered to stainless steel jigs (10 x 10 mm²) with an Araldite (registered trademark) adhesive, and a tensile load was applied using a universal testing machine (AGS-H Autograph; Shimazu Corporation, Japan) at a cross-head speed of 1 mm·mm⁻¹ until breakage.

The titanium metal porous surface material had an adhesion strength of 13.7±2.0 MPa (the average of seven samples), the Ti-15Mo-5Zr-3Al porous surface material had an adhesion strength of 11.6±2.9 MPa (the average of six samples), and the Ti-12Ta-9Nb-3V-6Zr-O porous surface material had an adhesion strength of 12.6±2.3 MPa (the average of seven samples), each having calcium phosphate compound-containing fine particles precipitated in the pores and being obtained in Example 5 through Example 4. It is presumed that high adhesion strength was obtained because a greater interlocking effect was achieved between hydroxyapatite and the titanium metal or titanium alloy after sandblasting.

### Example 6: Application of a different type of abrasive

Porous surface materials were produced in the same manner as in the preceding Examples except that a different type of abrasive was used.

Porous surface materials were produced by two-step sandblasting as follows. A Ti-6Al-4V plate with a size of 15×10×1 mm³ (Nishimura Co.,Ltd., Japan) and a Ti-22V-4Al plate with a size of 15×10×1 mm³ (Futaku Precision Machinery Industry, Japan) were used as titanium alloy materials. A first sandblasting step (PNEUMA-BLASTER (registered trademark) SFC-2; Fuji Manufacturing, Japan) was performed at a pressure of 0.85 MPa using an alumina media having a weight average particle diameter of 14.0±1.0 µm (JISR6001 Particle Size of a Grinding Stone) to treat the surfaces of the Ti-6Al-4V plate and Ti-22V-4Al plate. Subsequently, a second sandblasting step was performed using an alumina media having a weight average particle diameter of 3.0±0.4 µm (JISR6001 Particle Size of a Grinding Stone) in the same manner as in the first step to treat the surfaces of the Ti-6Al-4V plate and Ti-22V-4Al plate. The thus-obtained Ti-6Al-4V plate and Ti-22V-4Al plate in which pores were formed were washed with acetone and distilled water using an ultrasonic cleaner and dried at room temperature.

### Example 7: Application of a different type of abrasive

The porous surface materials obtained in Example 6 were immersed in simulated body fluid (SBF) as described below, thereby producing porous surface materials having calcium phosphate compound-containing fine particles precipitated in pores. The SBF was prepared as follows. Reagents NaCl, NaHCO₃, KCl, K₂HPO₄·3H₂O, MgCl₂·6H₂O, CaCl₂, and Na₂SO₄ were dissolved in ultrapure water with the composition shown in Table 1 above.

The pH of the SBF was adjusted to pH 8.20 at 36.5°C using trishydroxymethylaminomethane. Similar to the above, the porous surface materials obtained in Example 6, i.e., the Ti-6Al-4V porous surface material and the Ti-22V-4Al porous surface material, were immersed in this SBF, and cold isostatic pressing (CIP-SI; Kobe Steel, Japan) was performed at a pressure of 200 MPa for 60 minutes to permeate the SBF in the pores of the porous surface materials. Subsequently, the porous surface materials in the SBF were directly heated by using electromagnetic induction at 3 kW for 180 minutes. Using this procedure, calcium phosphate compound-containing fine particles as apatite nuclei were precipitated in the pores of the Ti-6Al-4V porous surface material and Ti-22V-4Al porous surface material. The thus-obtained porous surface materials were washed with distilled water and dried at room temperature.

### Test Example 5: Surface observation of porous surface materials

Each porous surface material (produced using Ti-6Al-4V or Ti-22V-4Al), in which calcium phosphate compound-containing fine particles were precipitated in the pores, obtained in Example 7 through Example 6 (two-step sandblasting using an alumina abrasive) was observed using a scanning electron microscope (SEM; SU6600, Hitachi High-Technologies, Japan). Fig. 14 shows the results. The pores formed in the surface by sandblasting were observed.

### Test Example 6: of the bioactivity of the porous surface material, in which calcium phosphate compound-containing fine particles were precipitated in the pores

1. The bioactivity of each porous surface material, in which calcium phosphate compound-containing fine particles were precipitated in the pores, obtained in Example 7 was evaluated as follows. Each porous surface material, in which calcium phosphate compound-containing fine particles were precipitated in the pores, obtained in Example 7 was immersed in SBF (pH 7.40 at 36.5°C). The surface of each porous surface material having hydroxyapatite formed thereon was analyzed using thin film X-ray diffraction (TF-XRD; Rint 2500; Rigaku Corporation, Japan), a scanning electron microscope (SEM; SU6600, Hitachi High-Technologies, Japan), and energy-dispersive X-ray spectroscopy (EDX; XFlash (registered trademark) 5010; Bruker, U.S.A.).

### SEM and EDX observation results

Each porous surface material (produced using Ti-6Al-4V or Ti-22V-4Al), in which calcium phosphate compound-containing fine particles were precipitated in the pores, obtained in Example 7 through Example 6 was immersed in SBF for one day or seven days, followed by SEM observation. Figs. 15 to 16 show the results. After one-day immersion, the surface was confirmed to be entirely covered with needle-shaped hydroxyapatite crystals. After seven-day immersion, the SEM observation confirmed the growth of the hydroxyapatite crystals, and the EDX analysis confirmed an increase in the intensity of peaks of P and Ca. As shown in Figs. 15(c) and 16(c), pores were observed; however, these pores were actually formed due to the hydroxyapatite layer, and were not formed in the titanium alloy surface by sandblasting.

### Thin film X-ray diffraction results

Each porous surface material (produced using Ti-6Al-4V or Ti-22V-4Al), in which calcium phosphate compound-containing fine particles were precipitated in the pores, obtained in Example 7 through Example 6, was immersed in SBF for 1 to 7 days, followed by thin film X-ray diffraction analysis. Fig. 17 shows the results. After one-day immersion, precipitation of hydroxyapatite was suggested. As the immersion period became longer, more peaks of hydroxyapatite were detected with higher intensity. The combination of the thin film X-ray diffraction results, SEM observation results, and EDX observation results suggest that hydroxyapatite was induced from apatite nuclei to cover the entire surface.

2. The adhesion strength between the porous surface material and hydroxyapatite formed on the surface thereof was measured using a modified ASTM C-633 method (W. Lacefield, Hydroxylapatite coatings, in: L.L. Hench, J. Wilson (Eds.), An Introduction to Bioceramics, World Sci. Singapore, 1993, pp. 223-238). Both surfaces of the porous surface material were adhered to stainless steel jigs (10 x 10 mm²) with an Araldite (registered trademark) adhesive, and a tensile load was applied using a universal testing machine (AGS-H Autograph; Shimazu Corporation, Japan) at a cross-head speed of 1 mm·mm⁻¹ until breakage.

The Ti-6Al-4V porous surface material had an adhesion strength of 12.8±3.2 MPa (the average of five samples), and the Ti-22V-4Al porous surface material had an adhesion strength of 13.1±2.1 MPa (the average of five samples), each having calcium phosphate compound-containing fine particles precipitated in the pores and being obtained in Example 7 through Example 6. It is presumed that even when alumina was used as an abrasive, a greater interlocking effect was achieved between hydroxyapatite and the titanium alloy after sandblasting, resulting in high adhesion strength.

### Example 8: Comparison due to differences in sandblasting

The differences in bioactivity and adhesion strength due to the differences in sandblasting were compared. The differences due to five types of treatment methods, i.e., (1) single-step sandblasting, (2) two-step sandblasting (first step: a media with large particle diameter; second step: a media with large particle diameter), (3) two-step sandblasting (first step: a media with large particle diameter; second step: a media with small particle diameter), (4) two-step sandblasting (first step: a media with small particle diameter; second step: a media with large particle diameter), and (5) two-step sandblasting (first step: a media with small particle diameter; second step: a media with small particle diameter), were compared.

The following five types of porous surface materials were produced by single-step sandblasting or two-step sandblasting. Ti-15Mo-5Zr-3Al plates each having a size of 15×10×3 mm³ (Kobe Steel, Japan) were used as titanium alloy materials.
(1) A single-step sandblasting (PNEUMA-BLASTER (registered trademark) SFC-2; Fuji Manufacturing, Japan) was performed at a pressure of 0.85 MPa using a silicon carbide media having a weight average particle diameter of 14.0±1.0 µm (JISR6001 Particle Size of a Grinding Stone) to treat the surface of the Ti-15Mo-5Zr-3Al plate. Hereafter, this sample is referred to as TMZA14.
(2) A first sandblasting step (PNEUMA-BLASTER (registered trademark) SFC-2; Fuji Manufacturing, Japan) was performed at a pressure of 0.85 MPa using a silicon carbide media having a weight average particle diameter of 14.0±1.0 µm (JISR6001 Particle Size of a Grinding Stone) to treat the surface of the Ti-15Mo-5Zr-3Al plate. Subsequently, a second sandblasting step was performed using a silicon carbide media having a weight average particle diameter of 14.0±1.0 µm (JISR6001 Particle Size of a Grinding Stone) in the same manner as in the first step to treat the surface of the Ti-15Mo-5Zr-3Al plate. Hereafter, this sample is referred to as TMZA14-14.
(3) A first sandblasting step (PNEUMA-BLASTER (registered trademark) SFC-2; Fuji Manufacturing, Japan) was performed at a pressure of 0.85 MPa using a silicon carbide media having a weight average particle diameter of 14.0±1.0 µm (JISR6001 Particle Size of a Grinding Stone) to treat the surface of the Ti-15Mo-5Zr-3Al plate. Subsequently, a second sandblasting step was performed using a silicon carbide media having a weight average particle diameter of 3.0±0.4 µm (JISR6001 Particle Size of a Grinding Stone) in the same manner as in the first step to treat the surface of the Ti-15Mo-5Zr-3Al plate. Hereafter, this sample is referred to as TMZA14-3.
(4) A first sandblasting step (PNEUMA-BLASTER (registered trademark) SFC-2; Fuji Manufacturing, Japan) was performed at a pressure of 0.85 MPa using a silicon carbide media having a weight average particle diameter of 3.0±0.4 µm (JISR6001 Particle Size of a Grinding Stone) to treat the surface of the Ti-15Mo-5Zr-3Al plate. Subsequently, a second sandblasting step was performed using a silicon carbide media having a weight average particle diameter of 14.0±1.0 µm (JISR6001 Particle Size of a Grinding Stone) in the same manner as in the first step to treat the surface of the Ti-15Mo-5Zr-3Al plate. Hereafter, this sample is referred to as TMZA3-14.
(5) A first sandblasting step (PNEUMA-BLASTER (registered trademark) SFC-2; Fuji Manufacturing, Japan) was performed at a pressure of 0.85 MPa using a silicon carbide media having a weight average particle diameter of 3.0±0.4 µm (JISR6001 Particle Size of a Grinding Stone) to treat the surface of the Ti-15Mo-5Zr-3Al plate. Subsequently, a second sandblasting step was performed using a silicon carbide media having a weight average particle diameter of 3.0±0.4 µm (JISR6001 Particle Size of a Grinding Stone) in the same manner as in the first step to treat the surface of the Ti-15Mo-5Zr-3Al plate. Hereafter, this sample is referred to as TMZA3-3.

The thus-obtained Ti-15Mo-5Zr-3Al plates in which pores were formed were washed with acetone and distilled water using an ultrasonic cleaner and dried at room temperature.

### Example 9: Comparison due to differences in sandblasting

The porous surface materials obtained in Example 8 were immersed in simulated body fluid (SBF) as described below, thereby producing porous surface materials having calcium phosphate compound-containing fine particles precipitated in pores. The SBF was prepared as follows. Reagents NaCl, NaHCO₃, KCl, K₂HPO₄·3H₂O, MgCl₂·6H₂O, CaCl₂, and Na₂SO₄ were dissolved in ultrapure water with the composition shown in Table 1 above.

The pH of the SBF was adjusted to pH 8.10 at 36.5°C using trishydroxymethylaminomethane. Similar to the above, the Ti-15Mo-5Zr-3Al porous surface materials obtained in Example 8 were immersed in this SBF, and cold isostatic pressing (CIP-SI; Kobe Steel, Japan) was performed at a pressure of 200 MPa for 60 minutes to permeate the SBF in the pores of the porous surface materials. Subsequently, the porous surface materials in the SBF were directly heated by using electromagnetic induction at 3 kW for 180 minutes. Using this procedure, calcium phosphate compound-containing fine particles as apatite nuclei were precipitated in the pores of the Ti-15Mo-5Zr-3Al porous surface materials. The thus-obtained porous surface materials were washed with distilled water and dried at room temperature.

### Test Example 7: Surface observation of porous surface materials

Each porous surface material (produced using Ti-15Mo-5Zr-3Al), in which calcium phosphate compound-containing fine particles were precipitated in the pores, obtained in Example 9 through Example 8 was observed using a scanning electron microscope (SEM; SU6600, Hitachi High-Technologies, Japan). Fig. 18 shows the results. The pores formed in the surface by sandblasting were observed.

### Test Example 8: of the bioactivity of the porous surface material, in which calcium phosphate compound-containing fine particles were precipitated in the pores

1. The bioactivity of each porous surface material, in which calcium phosphate compound-containing fine particles were precipitated in the pores, obtained in Example 9 was evaluated as follows. Each porous surface material, in which calcium phosphate compound-containing fine particles were precipitated in the pores, obtained in Example 9 was immersed in SBF (pH 7.40 at 36.5°C). The surface of each porous surface material having hydroxyapatite formed thereon was analyzed using a scanning electron microscope (SEM; SU6600, Hitachi High-Technologies, Japan), and energy-dispersive X-ray spectroscopy (EDX; XFlash (registered trademark) 5010; Bruker, U.S.A.).

### SEM and EDX observation results

Each porous surface material (produced using Ti-15Mo-5Zr-3Al), in which calcium phosphate compound-containing fine particles were precipitated in the pores, obtained in Example 9 through Example 8 was immersed in SBF for one day, followed by SEM observation. Figs. 19 to 23 show the results. After one-day immersion, the surfaces of TMZA14, TMZA14-3, and TMZA3-3 were confirmed to be entirely covered with hydroxyapatite crystals. The surface of TMZA3-14 was confirmed to be partially covered with hydroxyapatite crystals after one-day immersion, but hydroxyapatite did not grow on the entire surface.

2. The adhesion strength between the porous surface material and hydroxyapatite formed on the surface thereof was measured using a modified ASTM C-633 method (W. Lacefield, Hydroxylapatite coatings, in: L.L. Hench, J. Wilson (Eds.), An Introduction to Bioceramics, World Sci. Singapore, 1993, pp. 223-238). Both surfaces of the porous surface material were adhered to stainless steel jigs (10 x 10 mm²) with an Araldite (registered trademark) adhesive, and a tensile load was applied using a universal testing machine (AGS-H Autograph; Shimazu Corporation, Japan) at a cross-head speed of 1 mm·mm⁻¹ until breakage.

TMZA14 had an adhesion strength of 3.8±1.6 MPa, TMZA14-3 had an adhesion strength of 13.7±2.0 MPa, TMZA3-14 had an adhesion strength of 3.8±1.6 MPa, and TMZA3-3 had an adhesion strength of 7.9±2.2MPa, each having calcium phosphate compound-containing fine particles precipitated in the pores and being obtained in Example 9 through Example 8. TMZA14-3 showed the highest adhesion strength. It is presumed that high adhesion strength was obtained because pores with complicated shapes were effectively formed in the surface by sandblasting with particles (14 µm) to form pores, followed by sandblasting with particles (3 µm) to form smaller pores, achieving greater interlocking effect.

### Example 10: Imparting bioactivity to various titanium alloys

Porous surface materials were produced by two-step sandblasting as follows. A titanium metal plate with a size of 15×10×2 mm³ (Kobe Steel, Japan), a Ti-15Mo-5Zr-3Al plate with a size of 15×10×3 mm³ (Kobe Steel, Japan), and a Ti-12Ta-9Nb-3V-6Zr-O plate with a size of 20×10×1 mm³ (Nissey, Japan) were used as titanium alloy materials. A first sandblasting step (PNEUMA-BLASTER (registered trademark) SFC-2; Fuji Manufacturing, Japan) was performed at a pressure of 0.85 MPa using a silicon carbide media having a weight average particle diameter of 14.0±1.0 µm (JISR6001 Particle Size of a Grinding Stone) to treat the surfaces of the titanium metal plate, Ti-15Mo-5Zr-3Al plate, and Ti-12Ta-9Nb-3V-6Zr-O plate. Subsequently, a second sandblasting step was performed using a silicon carbide media having a weight average particle diameter of 3.0±0.4 µm (JISR6001 Particle Size of a Grinding Stone) in the same manner as in the first step to treat the surfaces of the titanium metal plate, Ti-15Mo-5Zr-3Al plate, and Ti-12Ta-9Nb-3V-6Zr-O plate. The thus-obtained titanium metal plate, Ti-15Mo-5Zr-3Al plate, and Ti-12Ta-9Nb-3V-6Zr-O plate in which pores were formed were washed with acetone and distilled water using an ultrasonic cleaner and dried at room temperature.

### Example 11: Production of porous surface materials having calcium phosphate compound-containing fine particles precipitated in pores

The porous surface materials obtained in Example 10 were immersed in simulated body fluid (SBF) as described below, thereby producing porous surface materials having calcium phosphate compound-containing fine particles precipitated in pores. The SBF was prepared as follows. Reagents NaCl, NaHCO₃, KCl, K₂HPO₄·3H₂O, MgCl₂·6H₂O, CaCl₂ and Na₂SO₄ were dissolved in ultrapure water with the composition shown in Table 1 above.

The pH of the SBF was adjusted to pH 8.10 at 36.5°C using trishydroxymethylaminomethane. Similar to the above, the porous surface materials obtained in Example 10, i.e., the titanium metal porous surface material, the Ti-15Mo-5Zr-3Al porous surface material, and the Ti-12Ta-9Nb-3V-6Zr-O porous surface material, were immersed in this SBF, and cold isostatic pressing (CIP-SI; Kobe Steel, Japan) was performed at a pressure of 200 MPa for 60 minutes to permeate the SBF in the pores of the porous surface materials. Subsequently, the porous surface materials in the SBF were directly heated by using electromagnetic induction at 3 kW for 180 minutes. Using this procedure, calcium phosphate compound-containing fine particles as apatite nuclei were precipitated in the pores of the titanium metal porous surface material, the pores of the Ti-15Mo-5Zr-3Al porous surface material, and the pores of the Ti-12Ta-9Nb-3V-6Zr-O porous surface material. The thus-obtained porous surface materials were washed with distilled water and dried at room temperature.

### Test Example 9: Surface observation of porous surface materials

Each porous surface material (produced using titanium metal, Ti-15Mo-5Zr-3Al, or Ti-12Ta-9Nb-3V-6Zr-O), in which calcium phosphate compound-containing fine particles were precipitated in the pores, obtained in Example 11 through Example 10 (two-step sandblasting) was observed using a scanning electron microscope (SEM; SU6600, Hitachi High-Technologies, Japan), and energy-dispersive X-ray spectroscopy (EDX; XFlash (registered trademark) 5010; Bruker, U.S.A.). Fig. 24 shows the results. The pores formed in the surface by sandblasting were observed.

### Test Example 10: Bioactivity evaluation

Each porous surface material (produced using titanium metal, Ti-15Mo-5Zr-3Al, or Ti-12Ta-9Nb-3V-6Zr-O), in which calcium phosphate compound-containing fine particles were precipitated in the pores, obtained in Example 11 through Example 10 (two-step sandblasting) was immersed in SBF (pH 7.40, 36.5°C). As a result, apatite covered the metal surface within one day, showing bioactivity.

Each porous surface material (produced using titanium metal, Ti-15Mo-5Zr-3Al, or Ti-12Ta-9Nb-3V-6Zr-O), in which calcium phosphate compound-containing fine particles were precipitated in the pores, obtained in Example 11 through Example 10 (two-step sandblasting) was immersed in human blood plasma (treated with sodium citrate, produced by Cosmo Bio Co., Ltd.). As a result, apatite covered the metal surface within one day, showing bioactivity.

### Example 12: Formation of pores by sandblasting using an abrasive made of alumina

Porous surface materials were produced by two-step sandblasting as follows. A Ti-6Al-4V plate with a size of 15×10×1 mm³ (Nishimura Co.,Ltd., Japan) and a Ti-22V-4Al plate with a size of 15×10×1 mm³ (Futaku Precision Machinery Industry, Japan) were used as titanium alloy materials. A first sandblasting step (PNEUMA-BLASTER (registered trademark) SFC-2; Fuji Manufacturing, Japan) was performed at a pressure of 0.85 MPa using an alumina media having a weight average particle diameter of 14.0±1.0 µm (JISR6001 Particle Size of a Grinding Stone) to treat the surfaces of the Ti-6Al-4V plate and Ti-22V-4Al plate. Subsequently, a second sandblasting step was performed using an alumina media having a weight average particle diameter of 3.0±0.4 µm (JISR6001 Particle Size of a Grinding Stone) in the same manner as in the first step to treat the surfaces of the Ti-6Al-4V plate and Ti-22V-4Al plate. The thus-obtained Ti-6Al-4V plate and Ti-22V-4Al plate in which pores were formed were washed with acetone and distilled water using an ultrasonic cleaner and dried at room temperature.

### Example 13: Production of porous surface materials having calcium phosphate compound-containing fine particles precipitated in pores

The porous surface materials obtained in Example 12 were immersed in simulated body fluid (SBF) as described below, thereby producing porous surface materials having calcium phosphate compound-containing fine particles precipitated in pores. The SBF was prepared as follows. Reagents NaCl, NaHCO₃, KCl, K₂HPO₄·3H₂O, MgCl₂·6H₂O, CaCl₂, and Na₂SO₄ were dissolved in ultrapure water with the composition shown in Table 1 above.

The pH of the SBF was adjusted to pH 8.20 at 36.5°C using trishydroxymethylaminomethane. Similar to the above, the porous surface materials obtained in Example 12, i.e., the Ti-6Al-4V porous surface material and the Ti-22V-4Al porous surface material, were immersed in this SBF, and cold isostatic pressing (CIP-SI; Kobe Steel, Japan) was performed at a pressure of 200 MPa for 60 minutes to permeate the SBF in the pores of the porous surface materials. Subsequently, the porous surface materials in the SBF were directly heated by using electromagnetic induction at 3 kW for 180 minutes. Using this procedure, calcium phosphate compound-containing fine particles as apatite nuclei were precipitated in the pores of the Ti-6Al-4V porous surface material and Ti-22V-4Al porous surface material. The thus-obtained porous surface materials were washed with distilled water and dried at room temperature.

### Test Example 11: Surface observation of porous surface materials

Each porous surface material (produced using Ti-6Al-4V or Ti-22V-4Al), in which calcium phosphate compound-containing fine particles were precipitated in the pores, obtained in Example 13 through Example 12 (two-step sandblasting) was observed using a scanning electron microscope (SEM; SU6600, Hitachi High-Technologies, Japan), and energy-dispersive X-ray spectroscopy (EDX; XFlash (registered trademark) 5010; Bruker, U.S.A.). Fig. 25 shows the results. The pores formed in the surface by sandblasting were observed.

### Test Example 12: Bioactivity evaluation

Each porous surface material (produced using Ti-6Al-4V or Ti-22V-4Al), in which calcium phosphate compound-containing fine particles were precipitated in the pores, obtained in Example 13 through Example 12 (two-step sandblasting) was immersed in SBF (pH 7.40, 36.5 °C). As a result, apatite covered the metal surface within one day, showing bioactivity.

Each porous surface material (produced using Ti-6Al-4V or Ti-22V-4Al), in which calcium phosphate compound-containing fine particles were precipitated in the pores, obtained in Example 13 through Example 12 (two-step sandblasting) was immersed in human blood plasma (treated with sodium citrate, produced by Cosmo Bio Co., Ltd.). As a result, apatite covered the metal surface within one day, showing bioactivity.

## Claims

1. A material having pores in the surface, the material being obtainable by a method comprising sandblasting a hard material to form pores in the surface,
the method comprising a plurality of sandblasting steps and including:
(A) sandblasting with media having a particle size that is different from that used in the immediately preceding sandblasting step.

2. The material according to claim 1, wherein Step (A) is performed 1 to 3 times.

3. The material according to claim 1 or 2, wherein at least one of sandblasting steps performed as Step (A) and the sandblasting step performed immediately proceeding Step (A) are performed with media having a particle size of 1 to 50 µm.

4. The material according to any one of claims 1 to 3, wherein the particle size of the media used in Step (A) is 1.5 to 10 times or 0.1 to 0.67 that of the media used in the sandblasting step performed before Step (A).

5. The material according to any one of claims 1 to 4, wherein the sandblasting is performed using, as an abrasive, silicon carbide, alumina, diamond, or silica.

6. The material according to any one of claims 1 to 5, wherein the hard material is an organic polymer, a metal, a ceramic, or a complex thereof.

7. The material according to any one of claims 1 to 6, which is used as a medical or dental material.

8. The material according to any one of claims 1 to 7, wherein calcium phosphate compound-containing fine particles are adhered to the surface thereof.

9. The material according to any one of claims 1 to 7, which supports calcium phosphate compound-containing fine particles on a surface.

10. The material according to claim 8, wherein the method further comprises:
(B) contacting the material obtained in Step (A) to a solution comprising calcium ions and hydrogen phosphate ions; and
(C) precipitating calcium phosphate compound-containing fine particles in the surface of the material obtained in Step (B).

11. A method for producing a material having pores in the surface thereof, comprising subjecting a hard material to a plurality of sandblasting steps, the method including:
(A) sandblasting with media having a particle size that is different from that of media used in a sandblasting step performed immediately preceding Step (A).
